# EUROPEAN PATENT SPECIFICATION

(11) **EP 3 283 031 B1**
(45) Date of publication and mention of the grant of the patent: **19.12.2018**
(21) Application number: 16718131.2
(22) Date of filing: 12.04.2016
(51) Int. Cl.: A61F 13/49, A61F 13/15

(54) **METHODS FOR MAKING ABSORBENT ARTICLES WITH A DESIGN HAVING A DISCONTINUOUS REGION BETWEEN TWO COMPONENTS ARRANGED TO PROVIDE A CONTIGUOUS APPEARANCE**
VERFAHREN ZUR HERSTELLUNG ABSORBIERENDER ARTIKEL MIT EINEM DESIGN EINES NICHTKONTINUIERLICHEN BEREICHS ZWISCHEN ZWEI KOMPONENTEN, DIE ZUR BEREITSTELLUNG EINES ZUSAMMENHÄNGENDEN ERSCHEINUNGSBILDES ANGEORDNET SIND
PROCÉDÉ DE FABRICATION D'ARTICLES ABSORBANTS À MOTIFS COMPORTANT UNE ZONE DISCONTINUE ENTRE DEUX COMPOSANTS AGENCÉS DE FAÇON À SEMBLER CONTIGUS

(30) Priority: 14.04.2015 US 201562147006 P
(43) Date of publication of application: 21.02.2018
(73) Proprietor: The Procter & Gamble Company, Cincinnati, OH 45202 (US)
(72) Inventor: ZINK, Ronald, Joseph, II, Cincinnati, Ohio 45202 (US); GREENING, Nelson, Edward, II, Cincinnati, Ohio 45202 (US); SAUER, Linda, Ann, Cincinnati, Ohio 45202 (US); LITCHHOLT, John, Joseph, Cincinnati, Ohio 45202 (US); WAGNER, Jason, Ashley, Cincinnati, Ohio 45202 (US); WOLFE, Sarah, Nicole, Cincinnati, Ohio 45202 (US)
(74) Representative: Kremer, Véronique Marie Joséphine
(86) International application number: PCT/US2016/027017
(87) International publication number: WO 2016/168136

(56) References cited:
- WO-A1-2006/017718
- WO-A1-2010/141302
- WO-A1-2012/131502
- WO-A1-2015/134459
- WO-A1-2016/100501
- WO-A1-2016/100502

## Description

### FIELD OF THE INVENTION

The present disclosure relates to methods for manufacturing absorbent articles, and more particularly, to assembling absorbent articles having a design including: a first region extending across a first component, a second region extending across a second component, and a discontinuous region separating the first region from the second region.

### BACKGROUND OF THE INVENTION

Along an assembly line, diapers and various types of other disposable absorbent articles may be assembled by adding components to and/or otherwise modifying advancing, continuous webs of material. Webs of material and component parts used to manufacture diapers may include: backsheets, topsheets, absorbent cores, front and/or back ears, fastener components, and various types of elastic webs and components such as leg elastics, barrier leg cuff elastics, and waist elastics.

Some consumers may prefer purchasing absorbent articles, such as diapers, having various types of different graphic designs printed thereon. As such, continuous substrates of material having printed graphics may be converted into different components used to assemble the absorbent articles. During the assembly process, the substrates of material having the graphics printed thereon may be subjected to various process transformations, such as folding, bonding, trimming, and/or cutting.

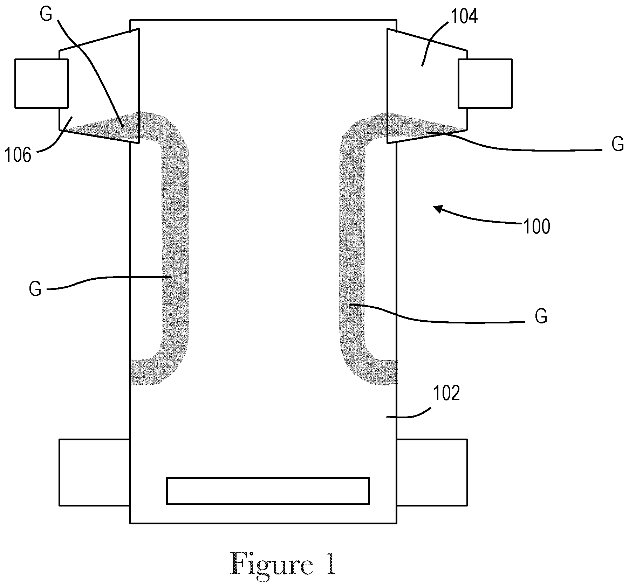

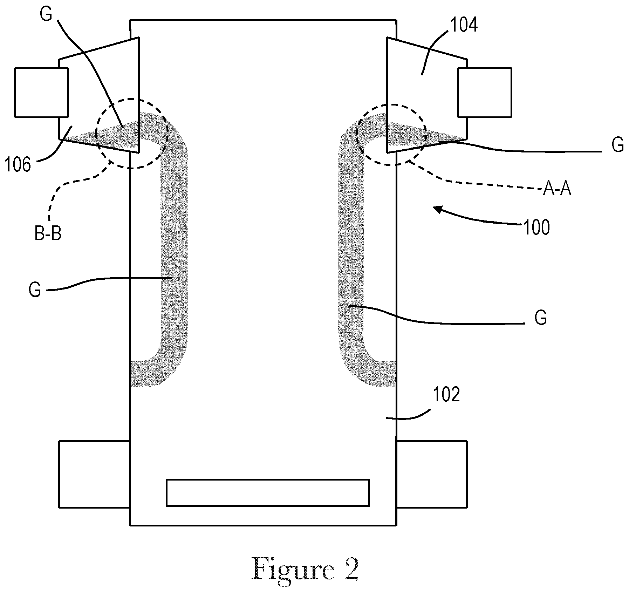

In some instances, consumers may prefer diapers with graphics defining various designs and various colored areas that may be printed thereon and that may extend over the entire area, or a relatively large area, of the diaper that is visible when worn. Thus, in converting operations involving the assembly of diapers having printed graphics that extend over relatively large regions, the printed substrates may be subjected to various process transformations in areas where the printing is located. However, subjecting printed substrates to various process transformations, such as folding, cutting, bonding, and/or assemblage with other printed components in areas where the graphics are located may create challenges in performing such process transformations when attempting to maintain aesthetically pleasing final assemblies. For example, imprecise and/or inconsistent bonding, cutting, and/or folding operations performed on a substrate in an area where a printed graphic is located may act to visibly highlight such process imprecisions or inconsistencies, such as crooked bond lines, fold lines, and/or cut lines. Some diapers are configured with designs intended to extend contiguously across multiple components that are assembled during the manufacturing process. However, imprecise placement of one printed component onto another printed component may be visibly highlighted when graphics on the separate components appear disjointed and/or misaligned when the components are combined. For example, Figure 1 shows an absorbent article 100 including examples of graphics G on assembled components, such as side panels 104, 106 and chassis 102, that require relatively precise alignment along the intersection of the side panels 104, 106 and the chassis 102 to provide the appearance of a contiguous design in the final assembly. And Figure 2, particularly in areas enclosed by circles A-A and B-B, illustrates how imprecise and/or inconsistent placement of the chassis 102 relative to the side panels 104, 106 during assembly results in the graphics G being disjointed. In addition, the aforementioned challenges may be exacerbated in absorbent article assembly processes operating at relatively high speed production rates (see e.g. WO 2012/131502 A1).

Consequently, there remains a need to incorporate substrates and/or components into absorbent article assembly processes wherein the substrates and/or components include graphics printed and/or positioned in such a manner so as to functionally reduce noticeable visible results of imprecise and/or inconsistent manufacturing operations performed in areas where the graphics are located.

### SUMMARY OF THE INVENTION

The present disclosure relates to absorbent articles and methods for assembling absorbent articles having a design including: a first graphic printed on a first component and a second graphic printed on a second component. During the assembly process, the first and second components are combined to form a discontinuous region devoid of printing and separating the first graphic from the second graphic. The discontinuous region may be defined by a substantially trapezoidal-shaped perimeter extending between an end edge of the first graphic and an end edge of the second graphic that defines an imaginary continuous extension of an established direction of the first graphic to the second graphic. Thus, the discontinuous region helps to provide the appearance that the first and second graphics form a contiguous design while at the same time mitigating the need to precisely align the graphics on separated components to form a contiguous design during the assembly process.

In one form, a method for assembling a disposable absorbent article comprises the steps of: providing a chassis having a longitudinal axis and a lateral axis, and having a first end region and a longitudinally opposing second end region separated from each other by a central region, the chassis comprising: an outer garment facing surface, an inner wearer facing surface, and an absorbent core disposed between the outer garment facing surface and the inner wearer facing surface, the chassis further comprising a first side edge and a second side edge separated laterally from the first side edge, the chassis comprising a printed chassis substrate comprising a first graphic comprising a longitudinal end edge extending longitudinally and adjacent a side edge of the printed chassis substrate, the longitudinal end edge of the first graphic comprising a length defined by a distance extending between a first side edge of the first graphic and a second side edge of the first graphic; providing a side panel comprising an outer lateral edge and an inner lateral edge, the side panel comprising a printed panel substrate comprising a second graphic, the second graphic comprising a lateral end edge adjacent a lateral edge of the printed panel substrate, the lateral end edge of the second graphic having a width defined by a distance extending laterally between a first side edge of the second graphic and a second side edge of the second graphic; and combining the first end region of the chassis and the side panel to define a discontinuous region devoid of printing and separating the first graphic from the second graphic, the discontinuous region defined by a substantially trapezoidal-shaped perimeter extending between the longitudinal end edge of the first graphic and the lateral end edge of the second graphic, defining an imaginary continuous extension of an established direction of the first graphic to the second graphic.

In another form, a method for assembling a disposable absorbent article comprises the steps of: providing a chassis having a longitudinal axis and a lateral axis, and having a first end region and a longitudinally opposing second end region separated from each other by a central region, the chassis comprising: a outer garment facing surface, an inner wearer facing surface, and an absorbent core disposed between the outer garment facing surface and the inner wearer facing surface, the chassis further comprising a first side edge and a second side edge separated laterally from the first side edge, a printed chassis substrate comprising a first graphic comprising a longitudinal end edge extending longitudinally and adjacent a side edge of the printed chassis substrate, the longitudinal end edge comprising a length defined by a distance extending between a first side edge of the first graphic and a second side edge of the first graphic; providing a belt comprising a first end region and a laterally opposing second region separated from each other by a central region, the belt comprising a printed panel substrate, the printed panel substrate comprising a second graphic, the second graphic comprising a lateral end edge adjacent a longitudinal edge of the printed panel substrate, the lateral end edge having a width defined by a distance extending laterally between a first side edge of the first graphic and a second side edge of the second graphic; connecting a fastening member with the first end region of the belt; combining the first end region of the chassis with the central region of the belt to define a discontinuous region devoid of printing and separating the first graphic from the second graphic, the discontinuous region defined by a substantially trapezoidal-shaped perimeter extending between the longitudinal end edge of the first graphic and the lateral end edge of the second graphic, defining an imaginary continuous extension of an established direction of the first graphic to the second graphic.

In yet another form, a method for assembling a disposable absorbent article comprises the steps of: providing a chassis having a longitudinal axis and a lateral axis, and having a first end region and a longitudinally opposing second end region separated from each other by a central region, the chassis comprising: a outer garment facing surface, an inner wearer facing surface, and an absorbent core disposed between the outer garment facing surface and the inner wearer facing surface, the chassis further comprising a first side edge and a second side edge separated laterally from the first side edge; providing a side panel comprising an outer lateral edge and an inner lateral edge, the side panel comprising a printed panel substrate comprising a first graphic, the first graphic comprising an end edge adjacent a longitudinal edge of the printed panel substrate, the end edge having a length defined by a distance extending laterally between a first side edge of the first graphic and a second side edge of the first graphic; providing a fastening member comprising a second graphic comprising a laterally extending end edge, the laterally extending end edge having a width defined by a distance extending laterally between a first side edge of the second graphic and a second side edge of the second graphic; combining the fastening member and the side panel to define a discontinuous region devoid of printing and separating the first graphic from the second graphic, the discontinuous region defined by a substantially trapezoidal-shaped perimeter extending between the end edge of the first graphic and the end edge of the second graphic, defining an imaginary continuous extension of an established direction of the first graphic to the second graphic; and combining the chassis and the side panel such that the side panel extends laterally outward from the first side edge of the first end region of the chassis.

In yet another form, a disposable absorbent article comprises: a chassis having a longitudinal axis and a lateral axis, and having a first end region and a longitudinally opposing second end region separated from each other by a central region, the chassis comprising: an outer garment facing surface, an inner wearer facing surface, and an absorbent core disposed between the outer garment facing surface and the inner wearer facing surface, the chassis further comprising a first side edge and a second side edge separated laterally from the first side edge, the chassis comprising a printed chassis substrate; a side panel connected with the first end region of the chassis, the side panel comprising an outer lateral edge and an inner lateral edge, the side panel comprising a printed panel substrate; and a design extending across a portion of the side panel and a portion of the chassis, the design comprising: a first region comprising a first graphic comprising a longitudinal end edge extending longitudinally and adjacent a side edge of the printed chassis substrate, the longitudinal end edge of the first graphic comprising a length defined by a distance extending between a first side edge of the first graphic and a second side edge of the first graphic; a second region comprising a second graphic, the second graphic comprising a lateral end edge adjacent a lateral edge of the printed panel substrate, the lateral end edge of the second graphic having a width defined by a distance extending laterally between a first side edge of the second graphic and a second side edge of the second graphic; and a discontinuous region devoid of printing and separating the first graphic from the second graphic, the discontinuous region defined by a substantially trapezoidal-shaped perimeter extending between the longitudinal end edge of the first graphic and the lateral end edge of the second graphic, defining an imaginary continuous extension of an established direction of the first graphic to the second graphic.

### BRIEF DESCRIPTION OF THE DRAWINGS

Figure 1 is a plan view of a diaper shown in a flat, uncontracted state and including graphics on side panels and chassis that require relatively precise alignment along the intersection of the side panels and the chassis to provide the appearance of a contiguous design.
Figure 2 is a view of the diaper of Figure 1 illustrating the resulting disjointed graphics caused by imprecise and/or inconsistent placement of the side panels relative to the chassis during assembly.
Figure 3A is a partially cut away plan view of a taped diaper with the portion of the diaper that faces away from a wearer oriented towards the viewer.
Figure 3B is a plan view of the taped diaper of Figure 3A with the portion of the diaper that faces toward a wearer oriented towards the viewer.
Figure 3C is a partially cut away plan view of a taped diaper with the portion of the diaper that faces away from a wearer oriented towards the viewer and including rear side panels defined by opposing end portions of a continuous belt.
Figure 3D is a partially cut away plan view of a taped diaper with the portion of the diaper that faces away from a wearer oriented towards the viewer and including front side panels defined by opposing end portions of a continuous belt.
Figure 4 is a plan view of a taped diaper in a flat, uncontracted state and including a design extending across outer, garment facing surfaces of a rear side panel, a front side panel, and a chassis.
Figure 5A is a detailed view of an embodiment discontinuous region of the design shown in Figure 4 that is devoid of printing between the first rear side panel and the chassis.
Figure 5B is a detailed view of an embodiment discontinuous region of the design shown in Figure 4 that is devoid of printing between the first front side panel and the chassis.
Figure 6 is a plan view of a taped diaper in a flat, uncontracted state and including a design visible across inner, wearer facing surfaces of a rear side panel, a front side panel, and a chassis.
Figure 7 is a plan view of a taped diaper in a flat, uncontracted state and including a design extending across outer, garment facing surfaces of a fastening member, a rear side panel, a front side panel, and a chassis.
Figure 7A is a detailed view of an embodiment discontinuous region of the design shown in Figure 7 that is devoid of printing between the first rear side panel and the fastening member.
Figure 8 is a detailed view of the intersection of the chassis and first rear side panel once tension is removed from elastic material in the first rear side panel and/or chassis and has contracted.
Figure 9 is a detailed view of fastening members of an absorbent article connected with a first waist region of a chassis.
Figure 10 is a simplified side elevation schematic view showing a method for assembling absorbent articles.
Figure 11 is a side view of a package of absorbent articles showing the package width, and wherein the outer surface of the package is illustrated as transparent for purposes of clarity.

### DETAILED DESCRIPTION OF THE INVENTION

The following term explanations may be useful in understanding the present disclosure:
"Absorbent article" is used herein to refer to consumer products whose primary function is to absorb and retain soils and wastes. "Diaper" is used herein to refer to an absorbent article generally worn by infants and incontinent persons about the lower torso. The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).

An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "graphic" refers to printed areas of substrates. Graphics may include a color difference or transition of one or more colors and may define images or designs that are constituted by a figure (for example, a line(s), a symbol or character), or the like. A graphic may include an aesthetic image or design that can provide certain benefit(s) when viewed. A graphic may be in the form of a photographic image. A graphic may also be in the form of a 1-dimensional (1-D) or 2-dimensional (2-D) bar code or a quick response (QR) bar code. A graphic design is determined by, for example, the color(s) used in the graphic (individual pure ink or spot colors as well as built process colors), the sizes of the entire graphic (or components of the graphic), the positions of the graphic (or components of the graphic), the movements of the graphic (or components of the graphic), the geometrical shapes of the graphic (or components of the graphics), the number of colors in the graphic, the variations of the color combinations in the graphic, the number of graphics printed, the disappearance of color(s) in the graphic, and the contents of text messages in the graphic.

It is to be appreciated that all graphics discussed herein may be in various different forms, shapes, and/or sizes than those depicted herein. It is also to be appreciated that the graphics described herein may be configured to be different graphics, standard graphics, custom graphics, and/or personalized graphics. "Different in terms of graphic design" means that graphics are intended to be different when viewed by users or consumers with normal attentions. Thus, two graphics having a graphic difference(s) which are unintentionally caused due to a problem(s) or an error(s) in a manufacture process, for example, are not different from each other in terms of graphic design. "Standard" or "standardized" refers to graphics, products, and/or articles that have the same aesthetic appearance without intending to be different from each other. The term "custom" or "customized" refers to graphics, products, and/or articles that are changed to suit a small demographic, region, purchaser, customer, or the like. Custom graphics may be selected from a set of graphics. For example, custom graphics may include animal depictions selected from groups of animals, such as farm animals, sea creatures, birds, and the like. In other examples, custom graphics may include nursery rhymes and the like. In one scenario, custom products or articles may be created by a purchaser of such products or articles wherein the purchaser selects graphics for the articles or products from a set of graphics offered by a manufacturer of such articles or products. Custom graphics may also include "personalized" graphics, which may be graphics created for a particular purchaser. For example, personalized graphics may include a person's name alone or in combination with a design.

In addition, all graphics discussed herein may be printed so as to fade from a high intensity zone to a low intensity zone such as disclosed in U.S. Patent Application Nos. 62/093,620; 62/093,438; 62/093,452; 62/093,516; and 62/093,604, filed on December 18, 2014, all of which are incorporated herein by reference. As used herein, the term "fade" means a visible gradual change in color hue, brightness, lightness, chroma, and/or saturation, for example, when a graphic fades from an area having a relatively high print density to an area having a relatively low print density. Further, the graphics herein may also be configured to change, appear, and/or disappear during usage.

"Longitudinal" means a direction running substantially perpendicular from a waist edge to a longitudinally opposing waist edge of an absorbent article when the article is in a flat out, uncontracted state, or from a waist edge to the bottom of the crotch, i.e., the fold line, in a bi-folded article. Directions within 45 degrees of the longitudinal direction are considered to be "longitudinal." "Lateral" refers to a direction running from a longitudinally extending side edge to a laterally opposing longitudinally extending side edge of an article and generally at a right angle to the longitudinal direction. Directions within 45 degrees of the lateral direction are considered to be "lateral."

The term "substrate" is used herein to describe a material which is primarily two-dimensional (i.e., in an XY plane) and whose thickness (in a Z direction) is relatively small (i.e., 1/10 or less) in comparison to its length (in an X direction) and width (in a Y direction). Nonlimiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

The term "taped diaper" (also referred to as "open diaper") refers to disposable absorbent articles having an initial front waist region and an initial back waist region that are not fastened, pre-fastened, or connected to each other as packaged, prior to being applied to the wearer. A taped diaper may be folded about the lateral centerline with the interior of one waist region in surface to surface contact with the interior of the opposing waist region without fastening or joining the waist regions together. Example taped diapers are disclosed in various suitable configurations U.S. Patent Nos. 5,167,897, 5,360,420, 5,599,335, 5,643,588, 5,674,216, 5,702,551, 5,968,025, 6,107,537, 6,118,041, 6,153,209, 6,410,129, 6,426,444, 6,586,652, 6,627,787, 6,617,016, 6,825,393, and 6,861,571; and U.S. Patent Publication Nos. 2013/0072887 A1; 2013/0211356 A1; and 2013/0306226 A1.

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed or pre-fastened by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed).

The present disclosure relates to absorbent articles and methods for assembling absorbent articles having a design including: a first region extending across a first component, a second region extending across a second component, and a discontinuous region separating the first region from the second region. In some configurations, the first region may include a first graphic printed on a first component. And the second region may include a second graphic printed on a second component. During the assembly process, the first and second components are combined to form a discontinuous region devoid of printing and separating the first region from the second region. As discussed in more detail below, the discontinuous region may be defined by a substantially trapezoidal-shaped perimeter extending between an end edge of the first graphic and an end edge of the second graphic that defines an imaginary continuous extension of an established direction of the first graphic to the second graphic. Thus, the discontinuous region helps to provide the appearance that the first and second graphics form a contiguous design while at the same time mitigating the need to precisely align the graphics on separated components to form a contiguous design. As such, substrates and/or components to be incorporated into manufactured absorbent articles herein include graphics that are positioned and/or printed in such a manner so as to functionally reduce noticeable visible results of imprecise and/or inconsistent manufacturing operations performed in areas where the graphics are located.

As previously mentioned, the processes and apparatuses discussed herein may be used in the manufacture of different types of absorbent articles. To help provide additional context to the subsequent discussion of the assembly process, the following provides a general description of absorbent articles in the form of taped diapers that include belt substrates that may be assembled in accordance with the methods and apparatuses disclosed herein.

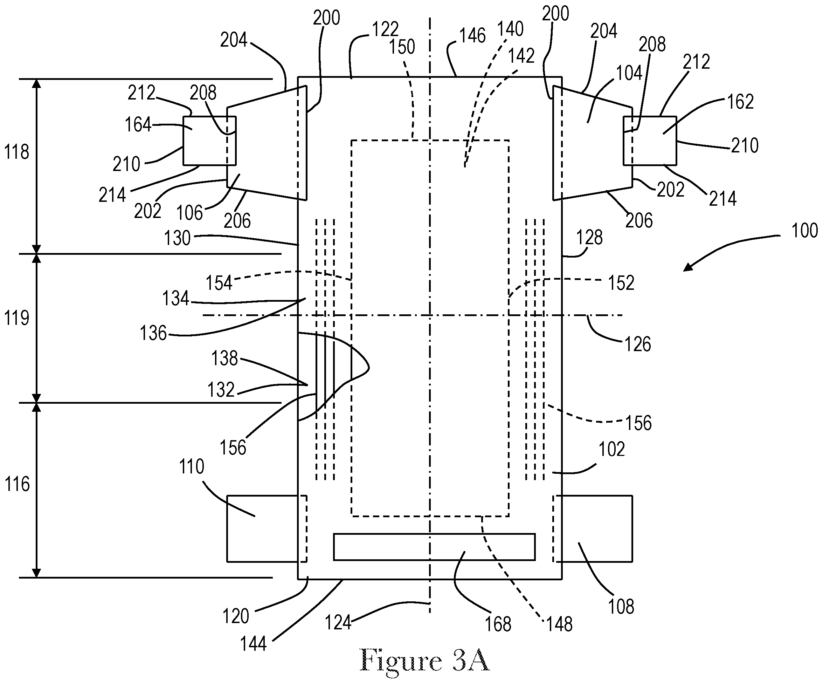

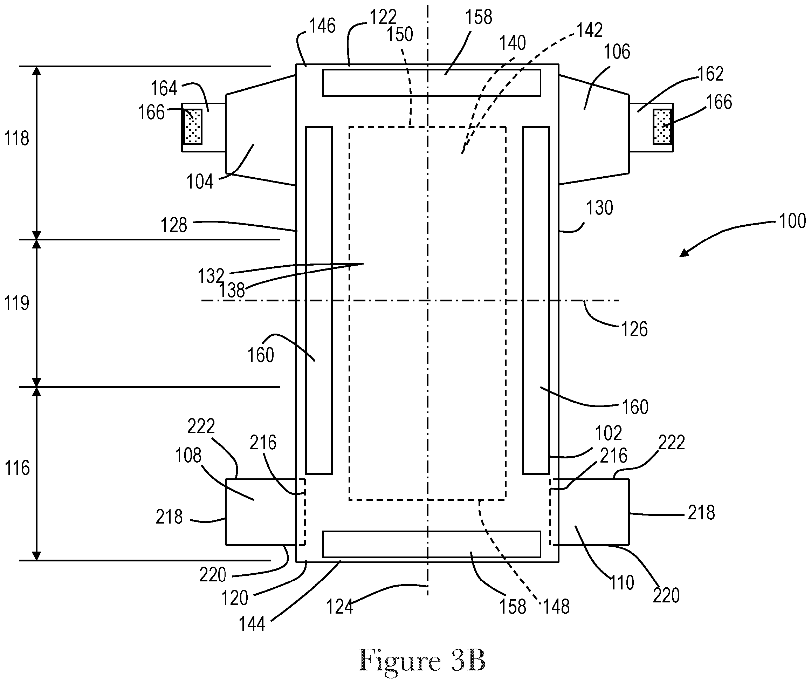

Figures 3A and 3B show an example of an absorbent article 100 that may be assembled in accordance with the methods disclosed herein. In particular, Figure 3A shows one example of a plan view of an absorbent article configured as a taped diaper 100, with the portion of the diaper that faces away from a wearer oriented towards the viewer. And Figure 3B shows a plan view of the diaper 100 with the portion of the diaper that faces toward a wearer oriented towards the viewer. The taped diaper 100 shown in Figures 3A and 3B includes a chassis 102, first and second rear side panels 104 and 106; and first and second front side panels 108 and 110.

As shown in Figures 3A and 3B, the diaper 100 and the chassis 102 each include a first waist region 116, a second waist region 118, and a crotch region 119 disposed intermediate the first and second waist regions. The first waist region 116 may be configured as a front waist region, and the second waist region 118 may be configured as back waist region. In some embodiments, the length of each of the front waist region, back waist region, and crotch region may be 1/3 of the length of the absorbent article 100. The absorbent article may also include a laterally extending front waist edge 120 in the front waist region 116 and a longitudinally opposing and laterally extending back waist edge 122 in the back waist region 118. To provide a frame of reference for the present discussion, the diaper 100 in Figures 3A and 3B are shown with a longitudinal axis 124 and a lateral axis 126. The longitudinal axis 124 may extend through a midpoint of the front waist edge 120 and through a midpoint of the back waist edge 122. And the lateral axis 126 may extend through a midpoint of a first longitudinal or right side edge 128 and through a midpoint of a second longitudinal or left side edge 130.

As shown in Figures 3A and 3B, the diaper 100 includes an inner, body facing surface 132, and an outer, garment facing surface 134. And the chassis 102 may include a backsheet 136 and a topsheet 138. The chassis 102 may also include an absorbent assembly 140, including an absorbent core 142, disposed between a portion of the topsheet 138 and the backsheet 136. As discussed in more detail below, the diaper 100 may also include other features, such as leg elastics and/or leg cuffs, an elastic waist region, and/or flaps, e.g., side panels and/or ears, to enhance the fits around the legs and waist of the wearer, to enhance the fit around the legs of the wearer.

As shown in Figures 3A and 3B, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in Figure 3A, the laterally extending end edges 144 and 146 may form a portion of the laterally extending front waist edge 120 in the front waist region 116 and a portion of the longitudinally opposing and laterally extending back waist edge 122 in the back waist region 118. When the diaper 100 is worn on the lower torso of a wearer, the front waist edge 120 and the back waist edge 122 may encircle a portion of the waist of the wearer. At the same time, the side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

It is to also be appreciated that a portion or the whole of the diaper 100 may also be made laterally extensible. The additional extensibility may help allow the diaper 100 to conform to the body of a wearer during movement by the wearer. The additional extensibility may also help, for example, the user of the diaper 100, including a chassis 102 having a particular size before extension, to extend the front waist region 116, the back waist region 118, or both waist regions of the diaper 100 and/or chassis 102 to provide additional body coverage for wearers of differing size, i.e., to tailor the diaper to an individual wearer. Such extension of the waist region or regions may give the absorbent article a generally hourglass shape, so long as the crotch region is extended to a relatively lesser degree than the waist region or regions, and may impart a tailored appearance to the article when it is worn.

As previously mentioned, the diaper 100 may include a backsheet 136. The backsheet 136 may also define the outer surface 134 of the chassis 102. The backsheet 136 may be impervious to fluids (e.g., menses, urine, and/or runny feces) and may be manufactured in part from a thin plastic film, although other flexible liquid impervious materials may also be used. The backsheet 136 may prevent the exudates absorbed and contained in the absorbent core from wetting articles which contact the diaper 100, such as bedsheets, pajamas and undergarments. The backsheet 136 may also comprise a woven or nonwoven material, polymeric films such as thermoplastic films of polyethylene or polypropylene, and/or a multi-layer or composite materials comprising a film and a nonwoven material (e.g., having an inner film layer and an outer nonwoven layer). The backsheet may also comprise an elastomeric film. An example backsheet 136 may be a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385. The backsheet 136 may also be embossed and/or matte-finished to provide a more clothlike appearance. Further, the backsheet 136 may permit vapors to escape from the absorbent core (i.e., the backsheet is breathable) while still preventing exudates from passing through the backsheet 136. The size of the backsheet 136 may be dictated by the size of the absorbent core 142 and/or particular configuration or size of the diaper 100.

Also described above, the diaper 100 may include a topsheet 138. The topsheet 138 may also define all or part of the inner surface 132 of the chassis 102. The topsheet 138 may be compliant, soft feeling, and non-irritating to the wearer's skin. It may be elastically stretchable in one or two directions. Further, the topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art.

Topsheets 138 may be selected from high loft nonwoven topsheets, apertured film topsheets and apertured nonwoven topsheets. Apertured film topsheets may be pervious to bodily exudates, yet substantially non-absorbent, and have a reduced tendency to allow fluids to pass back through and rewet the wearer's skin. Exemplary apertured films may include those described in U.S. Patent Nos. 5,628,097; 5,916,661; 6,545,197; and 6,107,539.

As mentioned above, the diaper 100 may also include an absorbent assembly 140 that is joined to the chassis 102. As shown in Figures 3A and 3B, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprises primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 and 2004/0097895.

As previously mentioned, the diaper 100 may also include elasticized leg cuffs 156 and an elasticized waistband 158. It is to be appreciated that the leg cuffs 156 can be and are sometimes also referred to as leg bands, side flaps, barrier cuffs, elastic cuffs or gasketing cuffs. The elasticized leg cuffs 156 may be configured in various ways to help reduce the leakage of body exudates in the leg regions. Example leg cuffs 156 may include those described in U.S. Patent Nos. 3,860,003; 4,909,803; 4,695,278; 4,795,454; 4,704,115; and U.S. Patent Publication No. 2009/0312730 A1.

The elasticized waistband 158 may provide improved fit and containment and may be a portion or zone of the diaper 100 that may elastically expand and contract to dynamically fit a wearer's waist. The elasticized waistband 158 may extend longitudinally inwardly from the waist edges 120, 122 of the diaper toward the lateral edges 148, 150 of the absorbent core 142. The diaper 100 may also include more than one elasticized waistband 158, for example, having one waistband 158 positioned in the back waist region 118 and one waistband 158 positioned in the front wait region 116, although other embodiments may be constructed with a single elasticized waistband 158. The elasticized waistband 158 may be constructed in a number of different configurations including those described in U.S. Patent Nos. 4,515,595 and 5,151,092. In some embodiments, the elasticized waistbands 158 may include materials that have been "prestrained" or "mechanically prestrained" (subjected to some degree of localized pattern mechanical stretching to permanently elongate the material). The materials may be prestrained using deep embossing techniques as are known in the art. In some embodiments, the materials may be prestrained by directing the material through an incremental mechanical stretching system as described in U.S. Patent No. 5,330,458. The materials are then allowed to return to their substantially untensioned condition, thus forming a zero strain stretch material that is extensible, at least up to the point of initial stretching. Examples of zero strain materials are disclosed in U.S. Patent Nos. 2,075,189; 3,025,199; 4,107,364; 4,209,563; 4,834,741; and 5,151,092.

As shown in Figure 3B, the chassis 102 may include longitudinally extending and laterally opposing side flaps 160 that are disposed on the interior surface 132 of the chassis 102 that faces inwardly toward the wearer and contacts the wearer. Each side flap may have a proximal edge. The side flaps may also overlap the absorbent assembly 140, wherien the proximal edges extend laterally inward of the respective side edges of the absorbent assembly 152 and 154. In some configurations, the side flaps may not overlap the absorbent assembly. It is to be appreciated that the side flaps may be formed in various ways, such as for example, by folding portions of the chassis 102 laterally inward, i.e., toward the longitudinal axis 124, to form both the respective side flaps and the side edges 128 and 130 of the chassis 102. In another example, the side flaps may be formed by attaching an additional layer or layers to the chassis at or adjacent to each of the respective side edges and of the chassis. Each of the side flaps may be joined to the interior surface 132 of the chassis and/or the absorbent assembly in side flap attachment zones in the front waist region 116 and in side flap attachment zones in the back waist region 118. The side flaps may extend to the same longitudinal extent as the absorbent article or alternatively the side flaps may have a longitudinal extent that is less than the absorbent article.

Taped diapers may be manufactured and provided to consumers in a configuration wherein the front waist region and the back waist region are not fastened, pre-fastened, or connected to each other as packaged, prior to being applied to the wearer. For example, the taped diaper 100 may be folded about a lateral centerline with the interior surface 132 of the first waist region 116 in surface to surface contact with the interior surface 132 of the second waist region 118 without fastening or joining the waist regions together. The rear side panels 104 and 106 and/or the front side panels 108 and 110 may also be folded laterally inward toward the inner surfaces 132 of the waist regions 116 and 118.

The diaper 100 may also include various configurations of fastening elements to enable fastening of the front waist region 116 and the back waist region 118 together to form a closed waist circumference and leg openings once the diaper is positioned on a wearer. For example, as shown in Figures 3A and 3B, the diaper 100 may include first and second fastening members 162, 164, also referred to as tabs, connected with the first and second rear side panels 104, 106, respectively. The diaper may also include first and second front side panels 108, 110, that may or may not include fastening members.

Referring back to Figures 3A and 3B, each rear side panel 104, 106 may include an inner longitudinal side edge 200, an outer longitudinal side edge 202, an outer lateral side edge 204, and an inner lateral side edge 206. In addition, each fastening member 162, 164 may also include an inner longitudinal side edge 208, an outer longitudinal side edge 210, an outer lateral side edge 212, and an inner lateral side edge 214. As shown in Figure 3A, proximal regions adjacent the inner longitudinal side edges 200 of the rear side panels 104, 106 may be connected with the backsheet 136 of the chassis 102. In addition, proximal regions adjacent the inner longitudinal side edges 208 of the fastening members 162, 164 may be connected with distal regions of the rear side panels 104, 106 adjacent the outer longitudinal side edges 202. As shown in Figure 3B, each front side panel 108, 110 may include an inner longitudinal side edge 216, an outer longitudinal side edge 218, an outer lateral side edge 220, and an inner lateral side edge 222. As such, proximal regions adjacent the inner longitudinal side edges 216 of the front side panels 108, 110 may be connected with the topsheet 138 of the chassis 102.

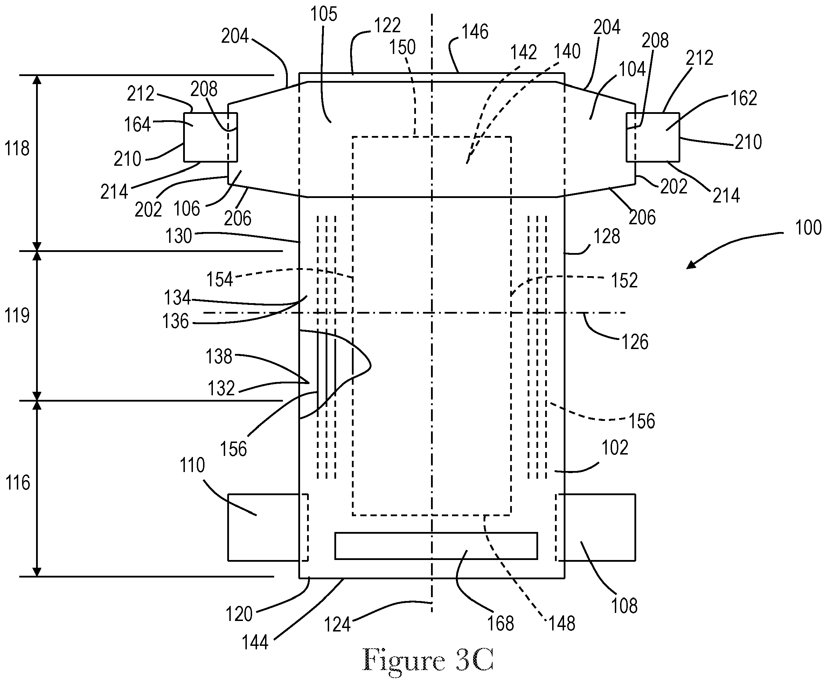

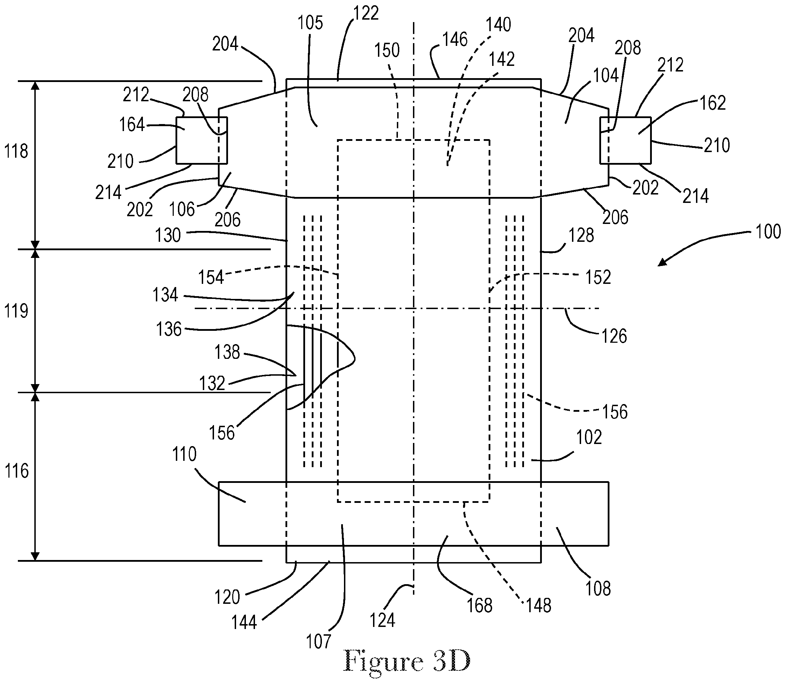

It is also to be appreciated that the rear side panels 104, 106 may be defined as discrete pieces and may also be defined by opposing end portions of a continuous belt 105, such as shown in Figure 3C. Similarly, the front side panels 108, 110 may be defined as discrete pieces and may also be defined by opposing end portions of a continuous belt 107, such as shown in Figure 3D. Examples of such belted configurations are disclosed in U.S. Patent Publication No. 2013/0306226 A1.

With continued reference to Figures 3A and 3B, each side panel 104, 106 and/or fastening member 162 and 164 may form a portion of or may be permanently bonded, adhered or otherwise joined directly or indirectly to the chassis 102 laterally inward from the side edge 128 and 130, in one of the front waist region 116 or the back waist region 118. Alternatively, the fastening members 162, 164 may form a portion of or may be permanently bonded, adhered or otherwise joined directly or indirectly to the first and second rear panels 104, 106 at or adjacent the distal edge of the panel and/or the first and second front side panels 108 and 110 at or adjacent the distal edge of the side panel. It is to be appreciated that the fastening members and/or side panels may be assembled in various ways, such as disclosed for example, in U.S. Patent No. 7,371,302. The fastening members 162, 164 and/or side panels 104, 106, 108, 110 may also be permanently bonded or joined at or adjacent the side edges 128 and 130 of the chassis 102 in various ways, such as for example, by adhesive bonds, sonic bonds, pressure bonds, thermal bonds or combinations thereof, such as disclosed for example, U.S. Patent No. 5,702,551.

It is to be appreciated that the rear side panels 104, 106 and/or the front side panels 108, 110 may comprise the same materials and/or may have the same structure. In some embodiments, the rear side panels 104, 106 and the front side panels 108, 110 may comprise different materials and/or may have different structures. It should also be appreciated that the rear side panels 104, 106 and the front side panels 108, 110 may be constructed from various materials. For example, the front and/or rear side panels may be manufactured from materials such as plastic films; apertured plastic films; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some embodiments, the front and/or rear side panels include a nonwoven web of synthetic fibers, and may include a stretchable nonwoven. In other embodiments, the front and/or side panels include an inner hydrophobic, non-stretchable nonwoven material and an outer hydrophobic, non-stretchable nonwoven material.

The rear side panels 104, 106 and/or the front side panels 108, 110 may also be elastic and may each include elastic material interposed between an outer substrate layer and the inner substrate layer. The elastic material may include one or more elastic elements such as strands, ribbons, films, or panels. In some configurations, the rear side panels 104, 106 and/or the front side panels 108, 110 may also define curved contours.

Referring now to Figure 3B, the first fastening member 162 and/or the second fastening member 164 may include various types of releasably engageable fasteners. The first and second fastening members 162 and/or 164 may also include various types of refastenable fastening structures. For example, the first and second fastening members 162 and 164 may include mechanical fasteners, 166, in the form of hook and loop fasteners, hook and hook fasteners, macrofasteners, buttons, snaps, tab and slot fasteners, tape fasteners, adhesive fasteners, cohesive fasteners, magnetic fasteners, hermaphrodidic fasteners, and the like. Some examples of fastening systems and/or fastening members 162, 164 are discussed in U.S. Patent Nos. 3,848,594; 4,662,875; 4,846,815; 4,894,060; 4,946,527; 5,151,092; 5,221,274; 6,251,097; 6,669,618; 6,432,098; and U.S. Patent Publication Nos. 2007/0078427 and 2007/0093769.

As previously mentioned, the fastening members 162 and 164 may be constructed from various materials and may be constructed as a laminate structure. The fastening members 162 and 164 may also be adapted to releasably and/or refastenably engage or connect with another portion of the diaper 100. For example, as shown in Figure 3A, the diaper 100 may include a connection zone 168, sometimes referred to as a landing zone, in the first waist region 116. As such, when the taped diaper 100 is placed on a wearer, the fastening members 162 and 164 may be pulled around the waist of the wearer and connected with the connection zone 168 in the first waist region 116 to form a closed waist circumference and a pair of laterally opposing leg openings. It is to be appreciated that the connection zone may be constructed from a separate substrate that is connected with the chassis 102 of the taped diaper. In some embodiments, the connection zone may be integrally formed as part of the backsheet 136 of the diaper 100 or may be formed as part of the first and second front panels 108, 110, such as described in U.S. Pat. Nos. 5,735,840 and 5,928,212.

As previously mentioned, the diaper 100 may include a design including: a first region extending across a first component, a second region extending across a second component, and a discontinuous region separating the first region from the second region. In some configurations, the first region may include a first graphic printed on a first component. And the second region may include a second graphic printed on a second component. During the assembly process, the first and second components are combined to form a discontinuous region devoid of printing and separating the first region from the second region. Thus, the graphics on the diaper components may be printed and/or positioned in such a manner so as to reduce noticeable visible results of imprecise and/or inconsistent assembly operations performed in areas where the graphics are located. As such, discontinuous regions may be positioned in locations that are subject to combining transformations during the assembly process, such as locations adjacent intersections between side panel edges and chassis side edges.

It is to be appreciated that the graphics described herein may be printed in various ways and may be printed by various types of printing accessories, such as ink jet, flexography, and/or gravure printing processes. Ink-jet printing is a non-impact dot-matrix printing technology in which droplets of ink are jetted from a small aperture directly to a specified position on a media to create a graphic. Two examples of inkjet technologies include thermal bubble or bubble jet and piezoelectric. Thermal bubble uses heat to apply to the ink, while piezoelectric uses a crystal and an electric charge to apply the ink. In some configurations, the printing stations may include a corona treater, which may be positioned upstream of the printer. The corona treater may be configured to increase the surface energy of the surface of the substrate to be printed. In some configurations, the printing stations may also include an ink curing apparatus. In some configurations, the ink curing apparatus may be in the form of an ultraviolet (UV) light source that may include one or more ultraviolet (UV) lamps, which may be positioned downstream of the printer to help cure inks deposited onto the substrate from the printer to form the graphics. In some configurations, the ink curing apparatus may also include an infrared (IR) dryer light source that may include one or more infrared (IR) lamps, which may be positioned downstream of the printer to help dry water-based or solvent-based inks deposited onto the substrate to form the graphics. In some configurations, the ink curing apparatus may include an electron beam (EB or e-beam) generator that may include one or more e-beam electrodes, which may be positioned downstream of the printer to help cure inks deposited onto the substrate from the printer to form the graphics. In some configurations, graphics may be created using pigments or dyes embedded in another fluid such as a glue or lotion, such as disclosed in U.S. Patent Publication No. 2014/0148773 A1.

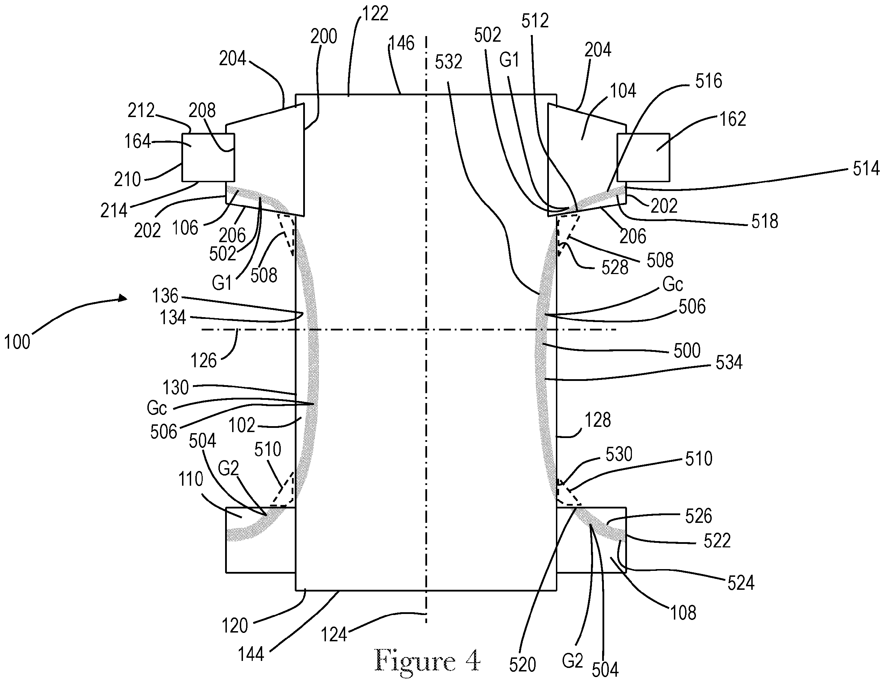

Figure 4 shows an example diaper 100 including a design 500 extending across the first rear side panel 104, the first front side panel 108, and the chassis 102. As shown in Figure 4, the design 500 may include a first region 502, a second region 504, a third region 506, a first discontinuous region 508, and a second discontinuous region 510. More particularly, the first region 502 comprises a first graphic G1 printed directly on the first rear side panel 104; the second region 504 comprises a second graphic G2 printed directly on the first front side panel 108; and the third region 506 comprises a chassis graphic Gc printed on the chassis 102. In turn, the first discontinuous region 508 separates the first region 502 from the third region 506 and is devoid of printing. And the second discontinuous region 510 separates the second region 504 from the third region 506 and is devoid of printing. As discussed above, the first rear side panel 104 and/or the first front side panel 108 may include one or more substrate layers. And it is to be appreciated that the first graphic G1 and/or the second graphic G2 may be printed on any of the substrate layers of the side panels 104, 108, and in turn, is referred to herein as printed panel substrates 600, 602. Similarly, as discussed above, the chassis 102 may include one or more substrate layers, such as the backsheet 136 and the topsheet 138. In addition, the backsheet 136 and/or topsheet 138 may include one or more substrate layers. For example, as discussed above, the backsheet 136 may include a nonwoven layer substrate and a film layer substrate. And it is to be appreciated that the chassis 102 may be printed on any of the substrate layers of the chassis 102, and in turn, is referred to herein as a printed chassis substrate 604.

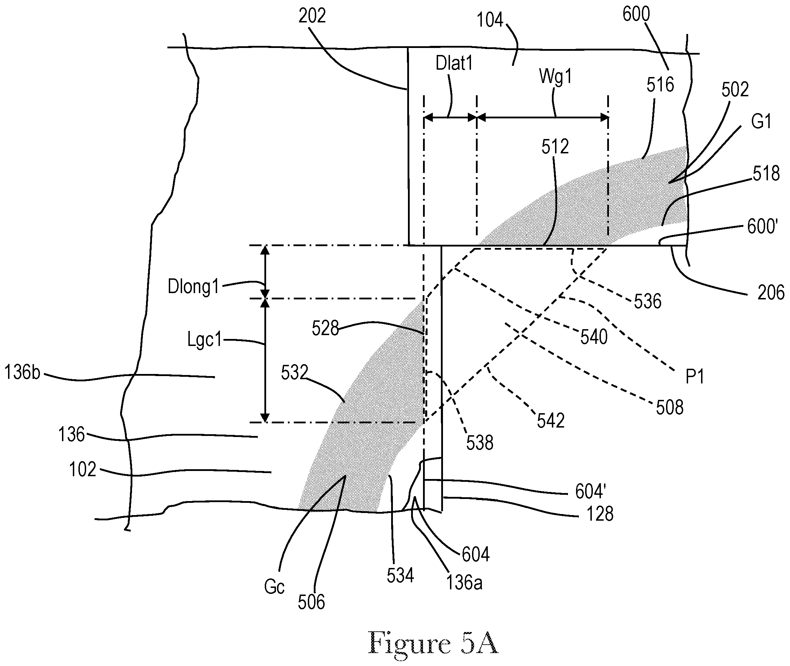

As shown in Figures 4 and 5A, the first graphic G1 defines a general stripe shape and extends from a first end edge 512 to a second end edge 514, and has a width Wg1 at the first end edge 512 defined by a distance extending laterally between a first side edge 516 and a second side edge 518 of the first graphic G1. In addition, the first side edge 516 of the first graphic G1 is laterally outboard relative the longitudinal axis 124 from a first side edge 604' of the printed chassis substrate 604 and laterally inboard relative the longitudinal axis 124 of the second side edge 518 of the first graphic G1. The first end edge 512 of the first graphic G1 extends between the first side edge 516 and the second side edge 518 adjacent an inner lateral end edge 600' of the printed panel substrate 600 of the first rear side panel 104, and the second end edge 514 of the first graphic G1 may be adjacent the outer longitudinal side edge 202 of the side panel 104. In some configurations, the first end edge 512 of the first graphic G1 may be positioned longitudinally outboard of the inner lateral end edge 206 of the side panel 104, and/or the second end edge 514 of the first graphic G1 may be positioned laterally inboard of the outer longitudinal side edge 202 of the side panel 104. In some configurations, the first end edge 512 of the first graphic G1 may be coextensive with a portion of the inner lateral end edge 206 of the side panel 104, and/or the second end edge 514 of the first graphic G1 may be coextensive with a portion of the outer longitudinal side edge 202 of the side panel 104. As mentioned above, the inner lateral edge 206 of the side panel 104 may include non-linear or curved portions, and as such, the first end edge 512 of the first graphic G1 may also be non-linear or curved. Also, in some configurations, the second side edge 518 of the first graphic G1 may be designed to provide the appearance of a shaped inner lateral edge 206.

As discussed above, the first graphic G1 is printed on the printed panel substrate 600 of the side panel 104. And it is to be appreciated that the first end edge 512 of the first graphic G1 may be located in various positions relative the inner lateral edge 600' of the printed panel substrate 600 and/or inner lateral edge 206 of the side panel 104. For example, as shown in Figure 5A, the first graphic G1 may be printed on an outer, garment facing substrate layer, which is also shown as the printed panel substrate 600 in Figure 5A. And the first end edge 512 of the first graphic G1 may be coextensive and coincident with a portion of the inner lateral end edge 600' of the printed panel substrate 600, and the inner lateral end edge 600' of the printed panel substrate 600 may also define the inner lateral edge 206 of the side panel 104. In some embodiments, the first end edge 512 of the first graphic G1 may be positioned longitudinally outboard of an inner lateral end edge 600' of the printed panel substrate 600. In particular, the first end edge 512 may be positioned longitudinally outboard of and separated from the inner lateral end edge 600' of the printed panel substrate 600. In some embodiments, the inner lateral edge 600' of the printed substrate 600 may also be positioned longitudinally outboard of the inner lateral edge 206 of the side panel 104.

With continued reference to Figures 3B, 4 and 5B, the second graphic G2 defines a general stripe shape and extends from a first end edge 520 to a second end edge 522, and has a width Wg2 at the first end edge 520 defined by a distance extending laterally between a first side edge 524 and a second side edge 526 of the second graphic G2. In addition, the first side edge 524 of the second graphic G2 is laterally outboard relative the longitudinal axis 124 from the first side edge 604' of the printed chassis substrate 604 and laterally inboard relative the longitudinal axis 124 of the second side edge 526 of the second graphic G2. The first end edge 520 of the second graphic G2 extends between the first side edge 524 and the second side edge 526 adjacent the inner lateral end edge 602' of the printed panel substrate 602 of the first front side panel 108, and the second end edge 526 of the second graphic G2 may be adjacent the inner longitudinal side edge 222 of the side panel 108. In some configurations, the first end edge 520 of the second graphic G2 may be positioned longitudinally outboard of the inner lateral end edge 222 of the side panel 108, and/or the second end edge 522 of the second graphic G2 may be positioned laterally inboard of the outer longitudinal side edge 218 of the side panel 108. In some configurations, the first end edge 520 of the second graphic G2 may be coextensive with a portion of the inner lateral end edge 222 of the side panel 108, and/or the second end edge 522 of the second graphic G2 may be coextensive with a portion of the outer longitudinal side edge 218 of the side panel 108. As mentioned above, the inner lateral edge 222 of the panel 108 may include non-linear or curved portions, and as such, the first end edge 520 of the second graphic G2 may also be non-linear or curved. Also, in some configurations, the second side edge 526 of the second graphic G2 may be designed to provide the appearance of a shaped inner lateral edge 222.

It is to be appreciated that the widths Wg1, Wg2 of the first graphic G1 and the second graphic G2 may vary. In addition, the widths Wg1, Wg2 of the first and second graphics G1, G2 may also be equal to each other. It is also to be appreciated that in some embodiments, the widths Wg1 and/or Wg2 may be the nominal width of a printed line.

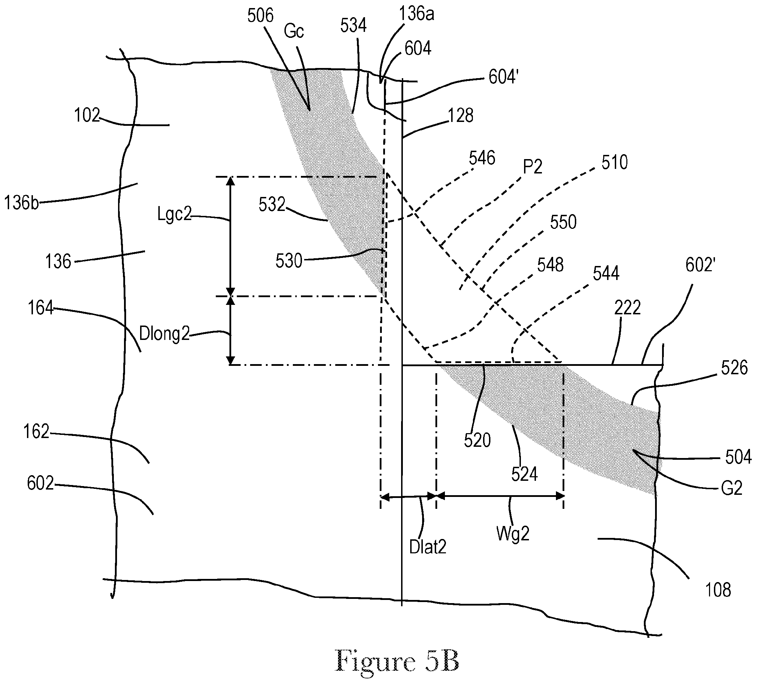

As discussed above, the second graphic G2 is printed on the printed panel substrate 602 of the first front side panel 108. And it is to be appreciated that the first end edge 520 of the second graphic G2 may be located in various positions relative the inner lateral edge 602' of the printed panel substrate 602 and/or inner lateral edge 222 of the side panel 108. For example, as shown in Figure 5B, the second graphic G2 may be printed on an outer, garment facing substrate layer, which is also shown as the printed panel substrate 602 in Figure 5B. And the first end edge 520 of the second graphic G2 may be coextensive and coincident with a portion of the inner lateral end edge 602' of the printed panel substrate 602, and the inner lateral end edge 602' of the printed panel substrate 602 may also define the inner lateral edge 222 of the side panel 108. In some embodiments, the first end edge 520 of the second graphic G2 may be positioned longitudinally outboard of an inner lateral end edge 602' of the printed panel substrate 602. In particular, the first end edge 520 is positioned longitudinally outboard of and separated from the inner lateral end edge 602' of the printed panel substrate 602. In some embodiments, the inner lateral edge 602' of the printed substrate 602 may also be positioned longitudinally outboard of the inner lateral edge 222 of the side panel 108.

Referring again to Figures 4, 5A, and 5B, the chassis graphic Gc defines a general stripe shape and extends longitudinally along the chassis 102 from a first end edge 528 to a second end edge 530. As shown in Figure 4, 5A, and 5B, the first end edge 528 extends between a first side edge 532 and a second side edge 534. As such, the chassis graphic Gc defines a length Lgc1 at the first end edge 528 defined by a distance extending longitudinally between the first side edge 532 and the second side edge 534. In addition, the first side edge 532 of the chassis graphic Gc is longitudinally inboard relative the lateral axis 126 from the inner lateral edge 600' of the printed panel substrate 600 of the first rear side panel 104 and longitudinally outboard relative the lateral axis 126 of the second side edge 534 of the chassis graphic Gc. As shown in Figure 5B, the second end edge 530 extends between the first side edge 532 and the second side edge 534. As such, the chassis graphic Gc defines a length Lgc2 at the second end edge 530 defined by a distance extending longitudinally between the first side edge 532 and the second side edge 534. In addition, the second side edge 532 of the chassis graphic Gc is longitudinally inboard relative the lateral axis 126 from the inner lateral edge 602' of the printed panel substrate 602 of the first front side panel 108 and longitudinally outboard relative the lateral axis 126 of the second side edge 534 of the chassis graphic Gc.

In some configurations, the first end edge 528 and the second end edge 530 of the chassis graphic Gc may both extend between the first side edge 532 and the second side edge 534 adjacent the first longitudinal side edge 128 of the chassis 102. In some configurations, the first end edge 528 of the chassis graphic Gc may be positioned away from the first longitudinal side edge 128 of the chassis 102, and/or the second end edge 530 of the chassis graphic Gc may be positioned away from the first longitudinal side edge 128 of the chassis 102. For example, the first end edge 528 of the chassis graphic Gc may be positioned laterally inboard of and separated from the first longitudinal side edge 128 of the chassis 102, and/or the second end edge 530 of the chassis graphic Gc may be positioned laterally inboard of and separated from the first longitudinal side edge 128 of the chassis 102. It is also to be appreciated that in some embodiments, the first end edge 528 and/or the second end edge 530 of the chassis graphic Gc may be coextensive with a portion of the first longitudinal side edge 128 of the chassis 102.

As discussed above, the chassis graphic Gc is printed on the printed chassis substrate 604 of the chassis 102. And it is to be appreciated that the first end edge 528 of the chassis graphic Gc may be located in various positions relative the longitudinal edge 604' of the printed chassis substrate 604 and/or first longitudinal edge 128 of the chassis 102. As discussed above and as shown in Figures 5A and 5B, the chassis 102 include various substrate layers, one of which may be configured as the printed chassis substrate 604. For example, the backsheet 136 may include an inner substrate 136a, such as a film layer substrate, and an outer substrate 136b, such as a nonwoven layer substrate. As such, the chassis graphic Gc is printed on the printed chassis substrate 604, which may also be the inner substrate 136a of the backsheet 136. As shown in Figures 5A and 5B, the first end edge 528 and/or the second end edge 530 of the chassis graphic Gc may be coextensive and coincident with a portion of the longitudinal side edge 604' of the printed chassis substrate 604, and the longitudinal side edge 604' of the printed substrate 604 may also be positioned laterally inboard the first longitudinal side edge 128 of the chassis 102. As such, it is also to be appreciated that various chassis components and/or substrates thereof that are devoid of printing may extend laterally outboard of the longitudinal side edge 604' of the printed substrate 604 to the chassis side edge 128, such as for example, portions of a topsheet 138, backsheet 136, and/or leg cuffs 156. In some embodiments, the first end edge 528 and/or the second end edge 530 of the chassis graphic Gc may be positioned laterally inboard of a longitudinal side edge 604' of the printed chassis substrate 604. And In some embodiments, the longitudinal side edge 604' of the printed substrate 604 may also define the first longitudinal side edge 128 of the chassis 102.

It is also to be appreciated that the chassis graphics Gc may be configured in various different designs and sizes. For example, the lengths Lgc1, Lgc2 of the chassis graphic Gc may vary. In some embodiments, the length Lgc1 of the chassis graphic Gc may be equal to or substantially equal to the length Lgc2. In some embodiments, the length Lgc1 of the chassis graphic Gc may be equal to or substantially equal to the width Wg1 of the first graphic G1. And in some embodiments, the length Lgc1 of the chassis graphic may be equal to or substantially equal to the width Wg2 of the second graphic G2. In some embodiments, the lengths Lgc1, Lgc2 and/or widths Wg1, Wg2 may be different. In some embodiments, the widths Wg1, Wg2 and/or the lengths Lgc1, Lgc2 may be from about 4 mm to about 30 mm. Further, the chassis graphic Gc may be printed on various chassis components, such as the backsheet 136, and may be printed prior to or during assembly of the chassis components. And as discussed above, the chassis graphic Gc may be printed on a backsheet film layer that is subsequently covered by a nonwoven layer such that the chassis graphic Gc is visible through the nonwoven layer.

As discussed above and as shown in Figures 4 and 5A, the first region 502 of the design 500 is separated from the third region 506 by the first discontinuous region 508, which is devoid of printing. As shown in detail in Figure 5A, the first discontinuous region 508 may include a first side edge 536, a second side edge 538, a third side edge 540, and a fourth side edge 542. The first side edge 536 may be coextensive and conterminous with the first end edge 512 of the first graphic G1, and the second side edge 538 may be coextensive and coterminous with the first end edge 528 of the chassis graphic Gc. The third side edge 540 may extend from the intersection of the first end edge 512 and the first side edge 516 of the first graphic G1 to the intersection of the first end edge 528 and the first side edge 532 of the chassis graphic Gc. And the fourth side edge 542 may extend from the intersection of the first end edge 512 and the second side edge 518 of the first graphic G1 to the intersection of the first end edge 528 and the second side edge 534 of the chassis graphic Gc. As such, the first side edge 536, the second side edge 538, the third side edge 540, and the fourth side edge 542 of the first discontinuous region 508 of the design 500 may be connected together so as to define a substantially trapezoidal-shaped perimeter P1 devoid of printing. In turn, the substantially trapezoidal-shaped perimeter P1 may extend between the first end edge 512 of the first graphic G1 and the first end edge 528 of the chassis graphic Gc, defining an imaginary continuous extension of an established direction of the first graphic G1 to the chassis graphic Gc.

As discussed above and as shown in Figures 4 and 5B, the second region 504 of the design 500 is separated from the third region 506 by the second discontinuous region 510, which is devoid of printing. As shown in detail in Figure 5B, the second discontinuous region 510 may include a first side edge 544, a second side edge 546, a third side edge 548, and a fourth side edge 550. The first side edge 544 may be coextensive and conterminous with the first end edge 520 of the second graphic G2, and the second side edge 546 may be coextensive and coterminous with the second end edge 530 of the chassis graphic Gc. The third side edge 548 may extend from the intersection of the first end edge 520 and the first side edge 524 of the second graphic G2 to the intersection of the second end edge 530 and the first side edge 532 of the chassis graphic Gc. And the fourth side edge 550 may extend from the intersection of the first end edge 520 and the second side edge 526 of the second graphic G2 to the intersection of the second end edge 530 and the second side edge 534 of the chassis graphic Gc. As such, the first side edge 544, the second side edge 546, the third side edge 548, and the fourth side edge 550 of the first discontinuous region 510 of the design 500 may be connected together so as to define a substantially trapezoidal-shaped perimeter P2 devoid of printing. In turn, the substantially trapezoidal-shaped perimeter P2 may extend between the first end edge 520 of the second graphic G2 and the second end edge 530 of the chassis graphic Gc, defining an imaginary continuous extension of an established direction of the second graphic G2 to the chassis graphic Gc.

As previously mentioned, including first and/or second discontinuous regions 508, 510 as part of the design 500 help to provide the appearance that the first graphic G1, second graphic G2, and chassis graphic Gc on the assembled first rear side panel 104, first front side panel 108, and chassis 102 form a contiguous design. In addition, the first and/or second discontinuous regions 508, 510 of the design 500 help to mitigate the need during the manufacturing process to precisely align the graphics G1, G2, and Gc on the first rear side panel 104, first front side panel 108, and chassis 102 to form a contiguous design.

As shown in Figure 5A, the intersection of the first end edge 512 and the first side edge 516 of the first graphic G1 may be positioned laterally outboard with respect to the longitudinal axis 124 of the longitudinal side edge 604' of the printed chassis substrate 604 of the chassis 102 by a distance Dlat1. And the intersection of the first end edge 528 and the first side edge 532 of the chassis graphic Gc may be positioned longitudinally inboard with respect to the lateral axis 126 of the inner lateral end edge 600' of the printed panel substrate 600 of the first belt 106 by a distance Dlong1. Similarly, as shown in Figure 5B, the intersection of the first end edge 520 and the first side edge 524 of the second graphic G2 may be positioned laterally outboard with respect to the longitudinal axis 124 of the first longitudinal side edge 604' of the printed chassis substrate 604 of the chassis 102 by a distance Dlat2. And the intersection of the first end edge 528 and the first side edge 532 of the chassis graphic Gc may be positioned longitudinally inboard with respect to the lateral axis 126 inner lateral end edge 602' of the printed panel substrate 602 of the second belt 108 by a distance Dlong2. Thus, the distances Dlong1 and Dlong2 may allow for some inconsistent and/or imprecise longitudinal placement of the side panels 104, 108 relative to the chassis 102 during manufacture. Similarly, the distances Dlat1 and Dlat2 may allow for some inconsistent and/or imprecise lateral placement of the side panels 104, 108 relative to the chassis 102 during manufacture. It is to be appreciated that the distances Dlat1, Dlong1, Dlat2, Dlong2 may vary depending on various factors, such as the precision and/or consistency of the manufacturing processes used to assemble the side panels 104, 108 and chassis 102. In some embodiments, the distances Dlat1, Dlong1, Dlat2, and/or Dlong2 may be equal to or greater than zero and less than about 30 mm. Thus, in contrast to the example discussed above with reference to Figures 1 and 2, the design 500 with the first and/or second discontinuous regions 508, 510 and the distances Dlat1, Dlong1, Dlat2, and/or Dlong2 such as shown in Figures 4, 5A, and 5B help provide the ability to combine separate components in such a manner so as to functionally reduce noticeable visible results of imprecise and/or inconsistent manufacturing operations performed in areas where the graphics are located.

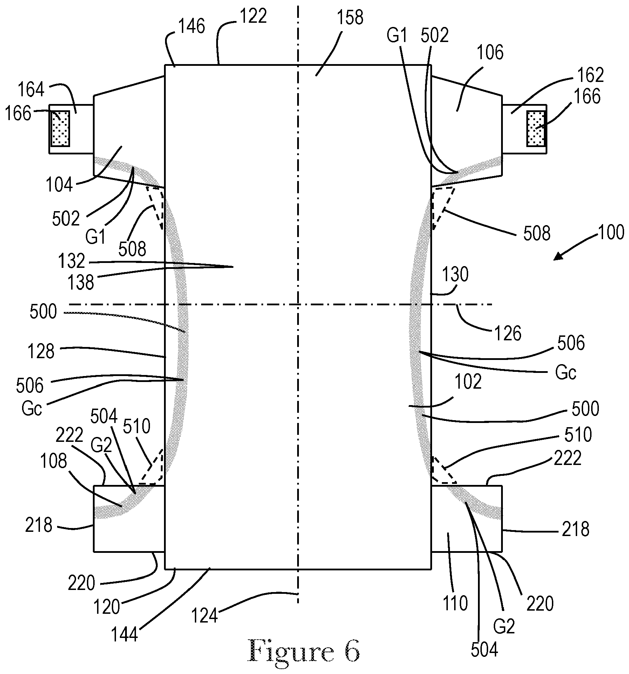

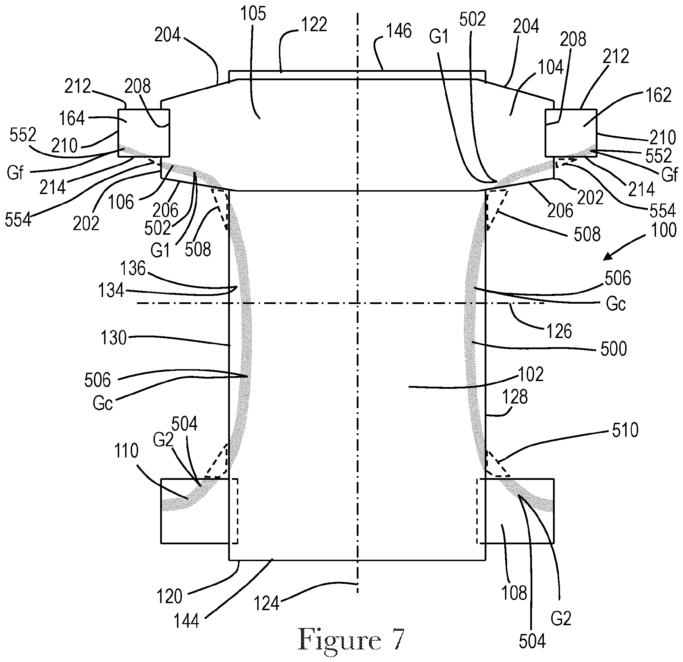

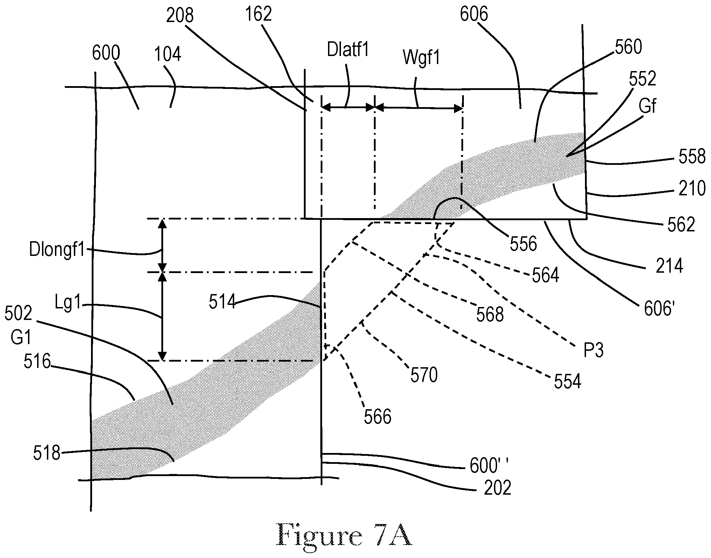

Although the above discussion is mainly provided in the context of the first graphic G1, the second graphic G2, and the chassis graphic Gc such as shown in Figure 4, it is to be appreciated that the diaper 100 may include various other graphics. For example, as shown in Figure 4, and as discussed below with reference to Figures 6 and 7, the diaper 100 may include additional graphics G1, G2, and Gc that are mirrored to the first graphic G1, the second graphic G2, and the chassis graphic Gc relative the longitudinal axis 124. In addition, the graphics may be positioned relative to each other on the various diaper components to provide designs with discontinuous regions and distances Dlat1 and Dlat2 that correspond with each other relative the longitudinal axis 124.

It is also to be appreciated that the graphics may be also visible when viewed from outer surfaces and/or inner surfaces of various substrates and components. For example, Figure 6 shows an absorbent article 100 with a design 500 similar to that described above with reference to Figures 4, 5A, and 5B, except the graphics G1, G2, and Gc are visible from the inner, wear facing surfaces 132 of the chassis 102, rear side panels 104, 106, and front side panels 106, 108. As such, the design 500 shown in Figure 6 extends across the first rear side panel 104, the first front side panel 108, and the chassis 102, and includes a first region 502, a second region 504, a third region 506, a first discontinuous region 508, and a second discontinuous region 510. And the first region 502 comprises a first graphic G1 printed directly on the first rear side panel 104; the second region 504 comprises a second graphic G2 printed directly on the first front side panel 108; and the third region 506 comprises a chassis graphic Gc printed on the chassis 102. In turn, the first discontinuous region 508 separates the first region 502 from the third region 506 and is devoid of printing. And the second discontinuous region 510 separates the second region 504 from the third region 506 and is devoid of printing. Thus, the discontinuous regions 508, 510, first graphics G1, second graphics G2, and chassis graphics Gc shown in Figure 6 may be configured with and/or have similar features as those described above with reference to Figures 4, 5A, and 5B. In addition, as discussed above, the first rear side panel 104 and/or the first front side panel 108 may include one or more substrate layers. And it is to be appreciated that printed panel substrates 600, 602 may be any of the substrate layers of the side panels 104, 108. For example, the first graphic and/or second graphic G2 may be printed an inner, wearer facing substrate layers of the side panels 104, 108. Similarly, as discussed above, it is to be appreciated that the printed chassis substrate 604 may comprise the topsheet 138 of the chassis 102. It also to be appreciated that an absorbent article 100 may be configured with graphics that are visible from both outer, garment facing surface and the inner, wearer facing surface, such as shown in Figure 4 and Figure 6.

It is also to be appreciated that the designs herein and associated graphics may be also configured with discontinuous regions between various other components. For example, Figure 7 shows an absorbent article 100 with a design 500 similar those described above and including a fourth region 552 extending across the fastening member 162. And the fourth region 552 comprises a fastener graphic Gf printed directly on the fastening member 162. In turn, a third discontinuous region 554 separates the first region 502 from the fourth region 552 on the side panel 104 and is devoid of printing. Although the rear side panels 104, 106 shown in Figure 7 are defined by opposing end portions of a continuous belt, it is to be appreciated that the absorbent article 100 and associated design 500 shown Figure 7 may be configured with discrete side panels 104, 106 such as discussed above with reference to Figures 3A, 3B, and 4. In addition, although the front side panels 108, 118 shown in Figure 7 are configured as discrete side panels, the it is to be appreciated that the absorbent article 100 and associated design 500 shown Figure 7 may be configured with front side panels 108, 110 defined by opposing end portions of a continuous belt such as discussed above with reference to Figure 3D. In addition, it is to be appreciated that the fastener graphic Gf may be printed on and/or visible from the inner, wearer facing side of the fastening member 162.

Figure 7A shows a detailed view of the fastener graphic Gf and third discontinuous region 554. It is to be appreciated that the fastening member 162 may include one or more substrate layers. And it is to be appreciated that the fastener graphic Gf may be printed on any of the substrate layers of the fastening member 162, and in turn, is referred to herein as printed fastener substrates 606. As shown in Figure 7A, the fastener graphic Gf defines a general stripe shape and extends from a first end edge 556 to a second end edge 558, and has a width at the first end edge 556 defined by a distance extending laterally between a first side edge 560 and a second side edge 562 of the fastener graphic Gf. In addition, the first side edge 560 of the fastener graphic Gf is laterally outboard relative the longitudinal axis 124 from a first side edge 600" of the printed panel substrate 600 and laterally inboard relative the longitudinal axis 124 of the second side edge 562 of the fastener graphic Gf. The first end edge 556 of the fastener graphic Gf extends between the first side edge 560 and the second side edge 562 adjacent an inner lateral end edge 606' of the printed fastener substrate 606 of the fastening member 162, and the second end edge 558 of the fastener graphic Gf may be adjacent the outer longitudinal side edge 210 of the fastening member 162. In some configurations, the first end edge 556 of the fastening graphic Gf may be positioned longitudinally outboard of the inner lateral end edge 214 of the fastening member 162, and/or the second end edge 558 of the fastener graphic Gf may be positioned laterally inboard of the outer longitudinal side edge 210 of the fastening member 162. In some configurations, the first end edge 556 of the fastener graphic Gf may be coextensive with a portion of the inner lateral end edge 214 of the fastening member 162, and/or the second end edge 558 of the fastener graphic Gf may be coextensive with a portion of the outer longitudinal side edge 210 of the fastening member 162. It is also to be appreciated that the inner lateral edge 214 of the fastening member may include non-linear or curved portions, and as such, the first end edge 556 of the fastener graphic Gf may also be non-linear or curved.

As discussed above, the fastener graphic Gf is printed on the printed fastener substrate 606 of the fastening member 162. And it is to be appreciated that the first end edge 556 of the fastener graphic Gf may be located in various positions relative the inner lateral edge 606' of the printed fastener substrate 606 and/or inner lateral edge 214 of the fastener member 162. For example, as shown in Figure 7A, the fastener graphic Gf may be printed on an outer, garment facing substrate layer, which is also shown as the printed fastener substrate 606 in Figure 7A. And the first end edge 556 of the fastener graphic Gf may be coextensive and coincident with a portion of the inner lateral end edge 606' of the printed fastener substrate 606, and the inner lateral end edge 606' of the printed fastener substrate 606 may also define the inner lateral edge 214 of the fastening member 162. In some embodiments, the first end edge 556 of the fastener graphic Gf may be positioned longitudinally outboard of an inner lateral end edge 606' of the printed fastener substrate 606. In particular, the first end edge 556 may be positioned longitudinally outboard of and separated from the inner lateral end edge 606' of the printed fastener substrate 606. In some embodiments, the inner lateral edge 606' of the printed fastener substrate 606 may also be positioned longitudinally outboard of the inner lateral edge 214 of the fastening member.

As discussed above and as shown in Figures 7 and 7A, the first region 502 of the design 500 is separated from the fourth region 552 by the third discontinuous region 554, which is devoid of printing. As shown in detail in Figure 7A, the third discontinuous region 554 may include a first side edge 564, a second side edge 566, a third side edge 568, and a fourth side edge 570. The first side edge 564 may be coextensive and conterminous with the first end edge 556 of the fastener graphic Gf, and the second side edge 566 may be coextensive and coterminous with the second end edge 514 of the first graphic G1. The third side edge 568 may extend from the intersection of the second end edge 514 and the first side edge 516 of the first graphic G1 to the intersection of the first end edge 556 and the first side edge 560 of the fastener graphic Gf. And the fourth side edge 570 may extend from the intersection of the second end edge 514 and the second side edge 518 of the first graphic G1 to the intersection of the first end edge 556 and the second side edge 562 of the fastener graphic Gf. As such, the first side edge 564, the second side edge 566, the third side edge 568, and the fourth side edge 570 of the third discontinuous region 554 of the design 500 may be connected together so as to define a substantially trapezoidal-shaped perimeter P3 devoid of printing. In turn, the substantially trapezoidal-shaped perimeter P3 may extend between the second end edge 514 of the first graphic G1 and the first end edge 556 of the fastener graphic Gf, defining an imaginary continuous extension of an established direction of the first graphic G1 to the fastener graphic Gf.

With continued reference to Figure 7A, the second end edge 514 of the first graphic G1 extends between the first side edge 516 and the second side edge 518. As such, the first graphic G1 defines a length Lg1 at the second end edge 514 defined by a distance extending longitudinally between the first side edge 516 and the second side edge 518. As also shown in Figure 7A, the fastener graphic Gf has a width Wgf1 at the first end edge 556 defined by a distance extending laterally between the first side edge 560 and the second side edge 562 of the fastener graphic Gf. In addition, the intersection of the first end edge 556 and the first side edge 560 of the fastener graphic Gf may be positioned laterally outboard with respect to the longitudinal axis 124 of the longitudinal side edge 600" of the printed panel substrate 600 by a distance Dlatf1. And the intersection of the second end edge 514 and the first side edge 516 of the first graphic G1 may be positioned longitudinally inboard with respect to the lateral axis 126 of the inner lateral end edge 606' of the printed fastener substrate 606 by a distance Dlongf1. Thus, the distances Dlongf1 and Dlatf1 may allow for some inconsistent and/or imprecise longitudinal placement of the side panels 104, 108 relative to the fastening members 162, 164 during manufacture. It is to be appreciated that the distances Dlatf1, Dlongf1 may vary depending on various factors, such as the precision and/or consistency of the manufacturing processes used to assemble the side panels 104, 108 and fastening members 162, 164. In some embodiments, the distances Dlatf1 and/or Dlongf1 may be equal to or greater than zero and less than about 30 mm.

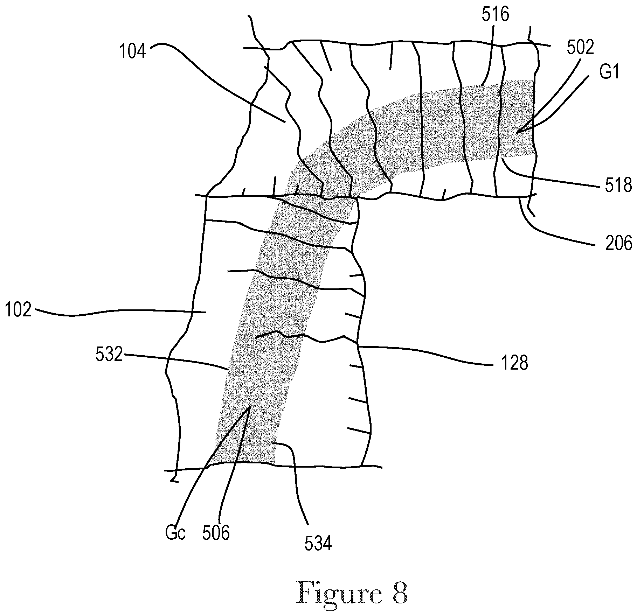

As discussed above, the rear side panels 104, 106 may include elastic material, such as elastic strands and/or film. In addition, the chassis 102 may include elastic material such as elasticized leg cuffs 156. During the assembly process, the elastic material in the first and/or second rear side panels 104, 106 and/or the chassis 102 may be positioned in an absorbent article 100 under tension and may be held under tension. However, such tension on the elastic material may be removed before or during the assembly process, allowing the elastic material in the rear side panels 104, 106 and/or the chassis 102 to contract. As such, contraction of the elastic material may deform the side panels 104, 106 and/or the chassis 102 so as to cause the first region 502, second region 504, and/or third region 506 of the design 500 to intersect or mate so as to form a contiguous design across the chassis 102 and the rear side panels 104, 106 and/or across the chassis 102 and the front side panels 108, 110. For example, Figure 8 shows a detailed view of the intersection of the chassis 102 and rear side panel 104 once tension is removed from elastic material in the side panel 104 and/or chassis 102 and has contracted. As shown in Figure 8, contraction of the elastic material may deform the chassis 102 and the side panel 104, causing the first graphic G1 and the chassis graphic Gc to mate and form a contiguous design extending across the chassis 102 and the side panel 104. It is to be appreciated that the first graphic G1 and the chassis graphic Gc may be mated in such a manner that the first discontinuous region 508, such as shown in Figure 5A, is partially or completely eliminated. In some configurations, the perimeter P1 of the first discontinuous region 508 may be deformed from a substantially trapezoidal shape to a substantially triangular shape. In some configurations, the first graphic G1 and the chassis graphic Gc may overlap one another. In addition, the first side edge 516 of the first graphic G1 and the first side edge 532 of the chassis graphic Gc may be aligned or offset from each other, and the second side edge 518 of the first graphic G1 and the first side edge 534 of the chassis graphic Gc may be aligned or offset from each other. Further contraction of the elastics can reduce the values of the dimensions of Dlat1 and/or Dlong1, referred to in Figure 5A, to zero.

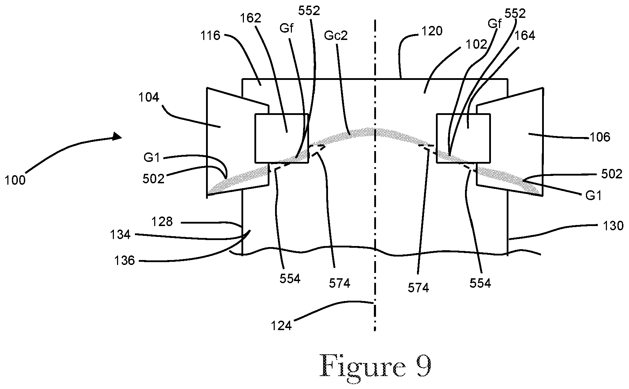

As discussed above with reference to Figures 3A and 3B, the fastening members 162 and 164 may be adapted to releasably and/or refastenably engage or connect with another portion of the diaper 100. For example, Figure 9 shows how the fasteners 162 and 164 may be pulled around the waist of the wearer and connected with the first waist region 116 of the diaper 100 to form a closed waist circumference and a pair of laterally opposing leg openings. As such, in some embodiments, the chassis may include a chassis graphic Gc2 positioned in the first waist region 116 that may be configured to provide the appearance that the chassis graphic Gc2 forms a contiguous design with graphics on other diaper components while at the same time mitigating the need to precisely align the graphics on other diaper components when placing the diaper 100 on a wearer's body. As shown in Figure 9, the chassis graphic Gc2 may be positioned such that when fastening members 162, 164 are connected with the first waist region 116, discontinuous regions 574 that is devoid of printing, such as described above, separate chassis the graphic Gc2 from the fastener graphics Gf.

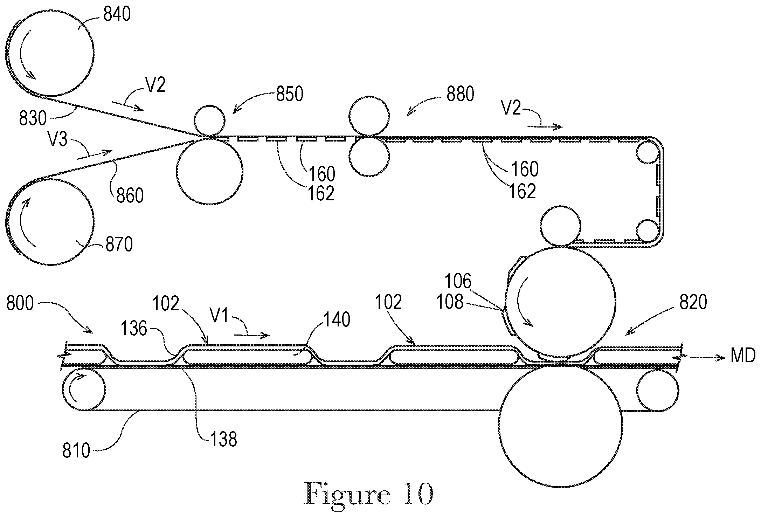

As previously mentioned, substrates and/or components that may be incorporated into manufactured absorbent articles, such as shown in Figures 3A-9, include graphics that may be positioned and/or printed in such a manner so as to reduce noticeable visible results of imprecise and/or inconsistent manufacturing operations performed in areas where the printing is located. It is to be appreciated that various apparatuses and methods may be utilized to assemble various components of diapers 100 described herein. For example, Figure 10 shows a simplified side elevation schematic view of a method for assembling absorbent articles 100. As shown in Figure 10, a continuous web 800 comprised of a plurality of interconnected chassis 102 are fed on conveyer 810 to first assembly station 820 at a first velocity V1. As discussed above, each chassis 102 may include a liquid pervious topsheet 138, a liquid impervious backsheet 136, and an absorbent core 140.

With continued reference to Figure 10, a side panel web 830 is unwound from supply roll 840 and fed to a second assembly station 850 at a second velocity, V2, wherein the second velocity V2 is less than the first velocity V1. In some embodiments, the side panel web 830 may include a laminate comprising a nonwoven web and a polymeric film. In some embodiments, the side panel web 830 may also include additional materials such as for example, nonwoven webs, polymeric films, elastomeric nonwovens, elastomeric films, elastomeric scrim, elastomeric foam, and the like. It is to be appreciated that one or more side panel webs 830 may be fed to the second assembly station 850. As shown in Figure 10, a fastening member substrate 860 may also unwound from supply roll 870 and fed to the second assembly station 850. The fastening member substrate 860 may be fed to the second assembly station 850 at a third velocity V3, wherein the third velocity is V3 is less than the second velocity V2. Having the second velocity V2 less than the first velocity, V1, and the third velocity V3 less than the second velocity V2 in the assembly configuration shown in Figure 10, helps enable the fastening member substrate 860 to be cut into individual fastening members 160, 162 and secured to the side panel web 830 in appropriate positions and also helps to allow the side panel web 830 to be cut into individual side panels 106, 108 (and/or belts 105) and secured to the chassis 102 in the appropriate positions.

At the second assembly station 850, the fastening member substrate 860 is cut into individual fastening members 160, 162 which may be bonded to the side panel web 830. It is to be appreciated that the fastening member substrate 860 may comprise various types of fastening member components, such as for example, adhesive tape substrates, mechanical fastener substrates, combination mechanical and adhesive fastener substrates, and the like. After the fastening member substrate 860 has been cut into individual fastening members 160, 162, the individual fastening members 160, 162 may be bonded to the side panel web 830 in various ways, such as for example, adhesive bonding, cohesive bonding, ultrasonic bonding, heat bonding, pressure bonding, friction bonding, autogenous bonding, and/or combinations of bonding methods. In some configurations, a pair of fastening member substrates 860 may be fed to the second assembly station 850 and cut into individual fastening members 160, 162 that are secured to either side is of the side panel web 830. The side panel web 830 having the individual fastening members 160, 162 bonded thereto may be slit into side panel ear webs at slitter 880 to provide a side panel 106, 108 on either side of the chassis 102.

With continued reference to Figure 10, the side panel webs 830 having the individual fastening members 160, 162 bonded thereto are then fed to the first assembly station 820 at the second velocity, V2. At the first assembly station 820 the side panel webs 830 having the securement members 160, 162 bonded thereto are cut into individual side panels 106, 108. The individual side panels 106, 108 are then bonded to the chassis 102 at the first assembly station 820 to form absorbent articles 100. It is to be appreciated that the individual side panels 106, 108 may be bonded to the chassis 102 in various ways, such as for example, adhesive bonding, cohesive bonding, ultrasonic bonding, heat bonding, pressure bonding, friction bonding, autogenous bonding, and/or combinations of bonding methods. It is also to be appreciated that the individual side panels 106, 108 may be bonded to the backsheet 136 and/or the topsheet 138. The individual side panels 106, 108 may also be inserted between the topsheet 138 and backsheet 136. The web 800 of interconnected chassis 102 having the side panels bonded thereto may then cut into individual disposable diapers 100. In some configurations, the web 800 of interconnected chassis 102 having the side panels 106, 108 bonded thereto may also be cut into individual disposable diapers 100 at the first assembly station 820.

As previously mentioned, various apparatuses and methods may be utilized to assemble various components of the diapers 100 described herein. For example, it is to be appreciated that the assembly configuration described above with reference to Figure 10 can be configured to also assemble the diapers 100 described above having rear side panels 104, 106; front side panels 108, 108; rear belts 105; and/or font belts 107. In addition to the example shown in Figure 10, other example assembly methods and apparatuses that may be utilized to assemble the diapers 100 described herein are disclosed in U.S. Patent Nos. 5,702,551 and 7,371,302; as well as U.S. Patent Publication No. 2013/0306226 A1.

In the context of the various components, designs, and graphics identified above for example, with reference to Figures 3A-9 as well as the assembly processes described above, a method for assembling such disposable absorbent articles 100 may include: providing a chassis 102 and providing a rear side panel 104 or a front side panel 108, wherein an end region 118 of the chassis 102 and the side panel 104 are combined to define a discontinuous region 508 devoid of printing and separating the first graphic G1 from the chassis graphic Gc, the discontinuous region 508 defined by a substantially trapezoidal-shaped perimeter P1 extending between the lateral end edge 512 of the first graphic G1 and the longitudinal end edge 528 of the chassis graphic Gc, defining an imaginary continuous extension of an established direction of the first graphic G1 to the chassis graphic Gc.

In another example, a method for assembling such disposable absorbent articles 100 may include: providing a chassis 102 and providing a belt 105, and connecting a fastening member 162 with an end region of a belt 105. The method may also include combining an end region 118 of the chassis 105 with a central region of the belt 105 to define a discontinuous region 508 devoid of printing and separating the first graphic G1 from the chassis graphic Gc, the discontinuous region 508 defined by a substantially trapezoidal-shaped perimeter P1 extending between the longitudinal end edge 528 of the chassis graphic Gc and the lateral end edge 512 of the first graphic G1, defining an imaginary continuous extension of an established direction of the first graphic G1 to the chassis graphic Gc.

In yet another example, a method for assembling such disposable absorbent articles 100 may include: providing: a chassis 102, a side panel 104, and a fastening member 162, wherein the fastening member 162 and the side panel 104 are combined to define a discontinuous region 554 devoid of printing and separating the first graphic G1 from the fastener graphic Gf, the discontinuous region 554 defined by a substantially trapezoidal-shaped perimeter P3 extending between the end edge 514 of the first graphic G1 and the end edge 556 of the fastener graphic Gf, defining an imaginary continuous extension of an established direction of the first graphic G1 to the fastener graphic Gf. In addition, the chassis 102 may be combined with the side panel 104 such that the side panel 104 extends laterally outward from the chassis 102.

### Packages

It is also to be appreciated that absorbent articles comprising graphics according to the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Accordingly, packages of the absorbent articles of the present disclosure may have an In-Bag Stack Height of less than about 110 mm, less than about 100 mm, less than about 80 mm, less than about 78 mm, or less than about 76 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 68 mm to about 110 mm or from about 72 mm to about 80 mm or from about 74 mm to about 78 mm, specifically reciting all 0.1 mm increments within the specified ranges and all ranges formed therein or thereby, according to the In-Back Stack Height Test described herein.

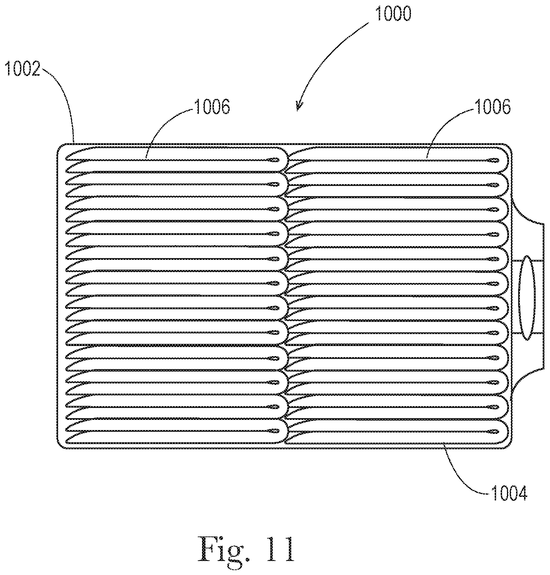

Figure 11 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

### In-Bag Stack Height Test

The in-bag stack height of a package of absorbent articles is determined as follows:

### Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within ± 0.5 mm. The horizontal sliding plate and the horizontal base plate are larger than the surface of the absorbent article package that contacts each plate, i.e., each plate extends past the contact surface of the absorbent article package in all directions. The horizontal sliding plate exerts a downward force of 850 ± 1 gram-force (8.34 N) on the absorbent article package, which may be achieved by placing a suitable weight on the center of the non-package-contacting top surface of the horizontal sliding plate so that the total mass of the sliding plate plus added weight is 850 ± 1grams.

### Test Procedure

Absorbent article packages are equilibrated at 23 ± 2 °C and 50 ± 5 % relative humidity prior to measurement.

The horizontal sliding plate is raised and an absorbent article package is placed centrally under the horizontal sliding plate in such a way that the absorbent articles within the package are in a horizontal orientation (see Figure 11). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) × 10 is calculated and reported to within ± 0.5 mm.

## Claims

1. A method for assembling a disposable absorbent article (100), the method comprising the steps of:
providing a chassis (102) having a longitudinal axis (124) and a lateral axis (126), and having a first end region (118) and a longitudinally opposing second end region (116) separated from each other by a central region (119), the chassis (102) comprising: an outer garment facing surface (134), an inner wearer facing surface (132), and an absorbent core (140) disposed between the outer garment facing surface (134) and the inner wearer facing surface (132), the chassis (102) further comprising a first side edge (128) and a second side edge (130) separated laterally from the first side edge (128), the chassis (102) comprising a printed chassis substrate (604) comprising a first graphic (Gc) comprising a longitudinal end edge (528) extending longitudinally and adjacent a side edge (604') of the printed chassis substrate (604), the longitudinal end edge (528) of the first graphic (Gc) comprising a length (Lgc1) defined by a distance extending between a first side edge (532) of the first graphic (Gc) and a second side edge (534) of the first graphic (Gc);
providing a side panel (104) comprising an outer lateral edge (202) and an inner lateral edge (200), the side panel (104) comprising a printed panel substrate (600) comprising a second graphic (G1), the second graphic (G1) comprising a lateral end edge (512) adjacent a lateral edge (600') of the printed panel substrate (600), the lateral end edge (512) of the second graphic (G1) having a width (Wg1) defined by a distance extending laterally between a first side edge (516) of the second graphic (G1) and a second side edge (518) of the second graphic (G1); and
combining the first end region (118) of the chassis (102) and the side panel (104) to define a discontinuous region (508) devoid of printing and separating the first graphic (Gc) from the second graphic (G1), the discontinuous region (508) defined by a substantially trapezoidal-shaped perimeter extending between the longitudinal end edge (528) of the first graphic (Gc) and the lateral end edge (512) of the second graphic (G1), defining an imaginary continuous extension of an established direction of the first graphic (Gc) to the second graphic (G1).

2. The method of claim 1, further comprising the steps of:
providing the chassis (102) under tension; and
removing the tension to form a contiguous design by mating the first graphic (Gc) and the second graphic (G1) upon contraction of the chassis (102).

3. The method according to any of the preceding claims, wherein the side panel (104) comprises a proximal region and a distal region laterally separated from the proximal region, and wherein the step of combining further comprises connecting the proximal region with the chassis (102).

4. The method according to any of the preceding claims, wherein the side panel (104) comprises a first end region and a laterally opposing second end region separated from each other by a central region, and the step of combining further comprises connecting the outer garment facing surface (134) of first end region (118) of the chassis (102) with the central region of the side panel.

5. The method according to any of the preceding claims, wherein the side panel (104) comprises elastic material.

6. The method according to any of the preceding claims, wherein the chassis (102) comprises a topsheet (138) defining the inner wearer facing surface (132), a backsheet (136) defining the outer garment facing surface (134), and wherein the absorbent core (140) is disposed between the topsheet (138) and the backsheet (136).

7. The method of claim 6, wherein the printed chassis substrate (604) comprises the topsheet (138).

8. The method of claim 6, wherein the printed chassis substrate (604) comprises the backsheet (136).

## Patentansprüche

1. Verfahren zum Zusammensetzen eines Einweg-Absorptionsartikels (100), wobei das Verfahren die folgenden Schritte umfasst:
Bereitstellen einer Außenhaut (102) mit einer Längsachse (124) und einer Querachse (126) und mit einem ersten Endbereich (118) und einem in Längsrichtung gegenüberliegenden zweiten Endbereich (116), die voneinander durch einen Zentralbereich (119) getrennt sind, wobei die Außenhaut (102) Folgendes umfasst: eine äußere bekleidungsseitige Oberfläche (134), eine innere trägerseitige Oberfläche (132), und einen Absorptionskern (140), der zwischen der der äußeren bekleidungsseitigen Oberfläche (134) und der inneren trägerseitigen Oberfläche (132) angeordnet ist, wobei die Außenhaut (102) ferner einen ersten Seitenrand (128) und einen zweiten Seitenrand (130), der lateral von dem ersten Seitenrand (128) getrennt ist, umfasst, wobei die Außenhaut (102) ein bedrucktes Außenhautsubstrat (604) umfasst, das eine erste Grafik (Gc) mit einem längs verlaufenden Endrand (528), der sich in Längsrichtung und benachbart zu einem Seitenrand (604') des bedruckten Außenhautsubstrates (604) erstreckt, umfasst, wobei der längs verlaufende Endrand (528) der ersten Graphik (Gc) eine Länge (Lgc1) umfasst, die durch einen Abstand zwischen einem ersten Seitenrand (532) der ersten Graphik (Gc) und einem zweiten Seitenrand (534) der ersten Graphik (Gc) definiert ist;
Bereitstellen eines Seitenfelds (104), das einen äußeren Seitenrand (202) und einen inneren Seitenrand (200) umfasst, wobei das Seitenfeld (104) ein bedrucktes Feldsubstrat (600) umfasst, das eine zweite Grafik (G1) umfasst, wobei die zweite Grafik (G1) einen seitlichen Endrand (512) benachbart zu einem Seitenrand (600') des bedruckten Feldsubstrates (600) umfasst, wobei der seitliche Endrand (512) der zweiten Grafik (G1) eine Breite (Wg1) aufweist, die durch einen Abstand definiert ist, der sich seitlich zwischen einem ersten Seitenrand (516) von der zweiten Graphik (G1) und einem zweiten Seitenrand (518) der zweiten Graphik (G1) erstreckt; und
Verbinden des ersten Endbereichs (118) der Außenhaut (102) mit dem Seitenfeld (104), um einen unterbrochenen Bereich (508) ohne Aufdruck zu definieren, und Trennen der ersten Grafik (Gc) von der zweiten Grafik (G1), wobei der unterbrochene Bereich (508) durch einen im Wesentlichen trapezförmigen äußeren Rand definiert ist, der sich zwischen dem längs verlaufenden Endrand (528) der ersten Grafik (Gc) und dem seitlichen Endrand (512) der zweiten Grafik (G1) erstreckt, und der eine gedachte ununterbrochene Ausdehnung einer festgelegten Richtung der ersten Grafik (Gc) zu der zweiten Grafik (G1) definiert.

2. Verfahren nach Anspruch 1, ferner umfassend die folgenden Schritte:
Bereitstellen der Außenhaut (102) unter Spannung; und
Entfernen der Spannung, um eine zusammenhängende Ausführung zu bilden, indem die erste Grafik (Gc) und die zweite Grafik (G1) beim Zusammenziehen der Außenhaut (102) zusammengefügt werden.

3. Verfahren nach einem der vorstehenden Ansprüche, wobei das Seitenfeld (104) einen proximalen Bereich und einen distalen Bereich, der seitlich von dem proximalen Bereich getrennt ist, umfasst, und wobei der Schritt des Kombinierens ferner das Verbinden des proximalen Bereichs mit der Außenhaut (102) umfasst.

4. Verfahren nach einem der vorstehenden Ansprüche, wobei das Seitenfeld (104) einen ersten Endbereich und einen seitlich gegenüberliegenden zweiten Endbereich umfasst, die durch einen Zentralbereich voneinander getrennt sind, und der Schritt des Verbindens ferner das Verbinden der äußeren bekleidungsseitigen Oberfläche (134) des ersten Endbereichs (118) der Außenhaut (102) mit dem Zentralbereich des Seitenfelds, umfasst.

5. Verfahren nach einem der vorstehenden Ansprüche, wobei das Seitenfeld (104) ein elastisches Material umfasst.

6. Verfahren nach einem der vorstehenden Ansprüche, wobei die Außenhaut (102) eine Oberschicht (138) umfasst, die die innere trägerseitige Oberfläche (132) definiert, eine Unterschicht (136), die die äußere bekleidungsseitige Oberfläche (134) definiert, und wobei der Absorptionskern (140) zwischen der Oberschicht (138) und der Unterschicht (136) angeordnet ist.

7. Verfahren nach Anspruch 6, wobei das bedruckte Außenhautsubstrat (604) eine Oberschicht (138) umfasst.

8. Verfahren nach Anspruch 6, wobei das bedruckte Außenhautsubstrat (604) eine Unterschicht (136) umfasst.

## Revendications

1. Procédé d'assemblage d'un article absorbant jetable (100), le procédé comprenant les étapes consistant à :
fournir un châssis (102) ayant un axe longitudinal (124) et un axe latéral (126), et ayant une première région d'extrémité (118) et une deuxième région d'extrémité longitudinalement opposée (116), séparées l'une de l'autre par une région centrale (119), le châssis (102) comprenant : une surface externe tournée vers le vêtement (134), une surface interne tournée vers le porteur (132) et une âme absorbante (140) disposée entre la surface externe tournée vers le vêtement (134) et la surface interne tournée vers le porteur (132), le châssis (102) comprenant en outre un premier bord latéral (128) et un deuxième bord latéral (130) séparé latéralement du premier bord latéral (128), le châssis (102) comprenant un substrat de châssis imprimé (604) comprenant un premier motif (Gc) comprenant un bord d'extrémité longitudinal (528) s'étendant longitudinalement et adjacent à un bord latéral (604') du substrat de châssis imprimé (604), le bord d'extrémité longitudinal (528) du premier motif (Gc) comprenant une longueur (Lgc1) définie par une distance s'étendant entre un premier bord latéral (532) du premier motif (Gc) et un deuxième bord latéral (534) du premier motif (Gc) ;
fournir un pan latéral (104) comprenant un bord latéral externe (202) et un bord latéral interne (200), le pan latéral (104) comprenant un substrat de pan imprimé (600) comprenant un deuxième motif (G1), le deuxième motif (G1) comprenant un bord d'extrémité latéral (512) adjacent à un bord latéral (600') du substrat de pan imprimé (600), le bord d'extrémité latéral (512) du deuxième motif (G1) ayant une largeur (Wg1) définie par une distance s'étendant latéralement entre un premier bord latéral (516) du deuxième motif (G1) et un deuxième bord latéral (518) du deuxième motif (G1) ; et
combiner la première région d'extrémité (118) du châssis (102) et le pan latéral (104) pour définir une région discontinue (508) dépourvue d'impression et séparant le premier motif (Gc) du deuxième motif (G1), la région discontinue (508) définie par un périmètre de forme essentiellement trapézoïdale s'étendant entre le bord d'extrémité longitudinal (528) du premier motif (Gc) et le bord d'extrémité latéral (512) du deuxième motif (G1), définissant une extension continue imaginaire d'une direction établie du premier motif (Gc) au deuxième motif (G1).

2. Procédé selon la revendication 1, comprenant en outre les étapes consistant à :
fournir le châssis (102) sous tension ; et
retirer la tension pour former un dessin contigu par appariement du premier motif (Gc) et du deuxième motif (G1) lors de la contraction du châssis (102).

3. Procédé selon l'une quelconque des revendications précédentes, dans lequel le pan latéral (104) comprend une région proximale et une région distale séparée latéralement de la région proximale, et dans lequel l'étape de combinaison comprend en outre le raccordement de la région proximale avec le châssis (102).

4. Procédé selon l'une quelconque des revendications précédentes, dans lequel le pan latéral (104) comprend une première région d'extrémité et une deuxième région d'extrémité latéralement opposée, séparées l'une de l'autre par une région centrale, et l'étape de combinaison comprend en outre le raccordement de la surface externe tournée vers le vêtement (134) de la première région d'extrémité (118) du châssis (102) avec la région centrale du pan latéral.

5. Procédé selon l'une quelconque des revendications précédentes, dans lequel le pan latéral (104) comprend un matériau élastique.

6. Procédé selon l'une quelconque des revendications précédentes, dans lequel le châssis (102) comprend une feuille de dessus (138) définissant la surface interne tournée vers le porteur (132), une feuille de fond (136) définissant la surface externe tournée vers le vêtement (134), et dans lequel l'âme absorbante (140) est disposée entre la feuille de dessus (138) et la feuille de fond (136).

7. Procédé selon la revendication 6, dans lequel le substrat de châssis imprimé (604) comprend la feuille de dessus (138).

8. Procédé selon la revendication 6, dans lequel le substrat de châssis imprimé (604) comprend la feuille de fond (136).
